# EUROPEAN PATENT APPLICATION

(11) **EP 4 545 555 A1**
(43) Date of publication of application: **30.04.2025**
(21) Application number: 23383100.7
(22) Date of filing: 27.10.2023
(51) Int. Cl.: C07K 14/47, A23J 1/20, A23J 3/08, A23L 33/10, A61K 38/01, A61K 38/17, A61P 35/00

(54) **RECOMBINANT PROTEINS AND THEIR USE IN CANCER THERAPY**

(71) Applicant: Real Deal Milk S.L., 08010 Barcelona (ES)
(72) Inventor: Oberlaender, Gabriel Mora, 08010 Barcelona (ES); Mirasol, Esther Paulo, 08010 Barcelona (ES); Ramos, Cristina Corral, 08010 Barcelona (ES); Mercado, Sergi Monforte, 08010 Barcelona (ES); Gilabert, Omar Tomás, 08010 Barcelona (ES); Toth-Czifra, Zoltan, 08010 Barcelona (ES)
(74) Representative: HGF

(57) **Abstract**

A recombinant protein for use in the treatment of a proliferative disease or disorder in a subject is provided, wherein the recombinant protein comprises at least one amino acid substitution or deletion relative to a corresponding native protein, and wherein the recombinant protein is not a hormone, an enzyme or an antibody.

## Description

### Field of the invention

The present invention relates to recombinant proteins, their production and their use in therapy. More particularly, the invention relates to recombinant proteins which can be used in the treatment of proliferative diseases, such as cancer.

### Background

The metabolism of cancer cells differs profoundly from that of healthy cells. Among other differences, enhanced energy demand and enhanced flow through biosynthetic pathways makes cancer cells strongly sensitive to environmental nutrient availability. Cancer cells have numerous and dramatic alterations in their DNA, resulting in complete absence of the information necessary to implement certain metabolic pathways or in inability to shift effectively between metabolic programs in response to environmental cues. In consequence, while healthy cells cope adequately with the absence or limitation of specific nutrients in the diet, cancer cells are unable to survive under the same conditions. This makes nutritional restriction an effective treatment against cancer, with much greater specificity than pharmacological treatment (chemotherapy). Furthermore, nutritional restriction is able to eliminate cells that are not effectively reached by the pharmacological treatment, e.g. due to poor vascularization, or that develop resistance to it, e.g. cancer stem cells. In addition to its own efficacy, nutritional restriction treatment can be applied in combination with other types of therapy.

One form of nutritional restriction treatment with proven efficacy against multiple types of cancer is amino acid (AA) restriction. Out of the 20 proteinogenic AAs, human cells are able to synthesize eleven. Elimination of these so-called non-essential amino acids (NEAA) from the diet is well tolerated by healthy cells. Cancerous cells, in contrast, might have lost the ability to synthesize one or some of these AA or might require them in larger amounts than what they can produce; absence of these AA from the diet is therefore lethal for these cells. The remaining nine proteinogenic AAs can not be synthesized by human cells, they must be consumed in the diet and are therefore called essential amino acids (EAA). Although prolonged EAA restriction is harmful to healthy cells, and therefore toxic to the organism, short periods of EAA starvation are tolerated by healthy cells. Cancer cells, due to their elevated biosynthetic requirements and inability to shift metabolic programs, are often unable to tolerate even this short periods of EAA starvation.

Beyond their indispensable role in protein synthesis, many amino acids have additional, complex, connections with other metabolic branches. An imbalance in their supply can therefore activate multiple failure points leading to cell death. The AAs serine and glycine and their basal role in one-carbon metabolism (sometimes referred to as serine-glycine-one-carbon (SGOC) metabolism) are a prime example of this. One-carbon metabolism comprises the folate and methionine cycles, which are responsible for the provision of one-carbon units for all methylation reactions and are therefore central to cellular function. Without a proper flow of carbon units through SGOC metabolism, the cell is unable to produce building blocks for lipid and DNA/RNA synthesis, making proliferation impossible. Besides biosynthetic functions, SGOC metabolism is required for methylation of DNA, histones and other proteins; DNA and protein methylation is fundamental to regulate expression and activity and its failure impedes proper cell operation. Furthermore, SGOC metabolism is one of the main regulators of redox balance and its failure leaves the cell very vulnerable to oxidative stress. The initial step in one-carbon metabolism is the methylation of tetra hydro folate (THF), which requires the side chain of serine as a one-carbon unit donor or, alternatively in some cells, the one-carbon unit produced by enzymatic cleavage of glycine. Given the fundamental role of SGOC metabolism in cell proliferation, it is not surprising that many cancer types show enhanced uptake of serine, overexpression of the enzymes involved in its synthesis or both, as well as sensitivity to dietary restriction of serine or combined restriction of serine and glycine. Defects of the protein p53 constitute an interesting biomarker as they predict extreme sensitivity to dietary serine restriction. The absence of dietary serine upregulates endogenous serine synthesis, which depletes glycolysis and forces the cancerous cell to increase flow through the TCA cycle. This, in turn, induces oxidative stress. In cells with functional p53 this shift is eventually reversed, limiting the extent of the oxidative challenge, although at the expense of reduced proliferation. Furthermore, because p53 defective cells are unable to slow down their cell cycle progression, use of the limited serine supply for nucleotide synthesis rather than for synthesis of the redox regulator glutathione is prioritized, making the oxidative challenge even more lethal. On the other hand, overexpression of the PHGDH gene (encoding phosphoglycerate dehydrogenase, a key enzyme in serine biosynthesis) regardless of an abundant supply of dietary serine is a marker predicting reduced efficacy of serine restriction. However, search for chemical inhibitors of PHGDH and other enzymes in the serine synthesis pathway is an active field of research where promising candidates have already been identified, and, in fact, the combination of chemotherapy against serine synthesis with dietary serine restriction has shown efficacy against cancers resistant to either therapy alone.

At present, two main approaches exist to produce a diet with AA restriction. The first is a strong reduction or complete exclusion of protein from the diet. While this has shown efficacy against disease progression in some cases, it is a risky approach as it results in very poor nutrition, weakens the patient and is not sustainable for prolonged periods. In fact, some dietary guidelines for cancer patients recommend high protein diets.

In the second approach, a formulation devoid of protein is supplemented with individual free amino acids, thereby excluding only those amino acids that are particularly necessary for the given type of cancer. This maximizes exploitation of the cancer's vulnerability while aiming to minimize the collateral impact on the patient's nutrition. This approach is still not ideal, as free AAs are not optimally absorbed and metabolized by the body, while spikes in blood AAs can drive alterations in blood glucose and insulin. In addition to these nutritional and metabolic disadvantages, a diet based on free AAs can result in gastrointestinal irritation. Furthermore, formulations based on free amino acids are not considered very palatable, negatively impacting the patient's general wellbeing and often resulting in poor adherence to the treatment.

The present invention has been devised with these issues in mind.

### Summary of the invention

According to a first aspect of the present invention, there is provided a recombinant protein comprising at least one amino acid substitution or deletion relative to a corresponding native protein.

According to a second aspect of the invention, there is provided a composition comprising the recombinant protein according to the first aspect of the invention. Optionally the composition is formulated for oral administration. The composition may be a nutritional formulation, for example a food formulation.

According to a third aspect of the invention, there is provided a recombinant protein according to the first aspect, or a composition according to the second aspect, for use in the treatment of a proliferative disease or disorder in a subject.

In some embodiments each residue of a first amino acid that is present in the corresponding native protein has been independently deleted or substituted for another amino acid in the recombinant protein. For example, it may be that each serine residue present in the native protein is independently deleted or substituted in the recombinant protein.

In some embodiments the recombinant protein is not a hormone, an enzyme or an antibody.

In some embodiments, the recombinant protein is a storage protein.

In some embodiments, the recombinant protein comprises all nine essential amino acids.

According to a fourth aspect of the invention, there is provided a nucleic acid molecule comprising a sequence encoding a recombinant protein as described herein.

According to a fifth aspect of the invention, there is provided a vector comprising the nucleic acid molecule of the fourth aspect of the invention.

According to a sixth aspect of the invention, there is provided a host cell comprising the nucleic acid molecule of the fourth aspect of the invention, or the vector of the fifth aspect of the invention.

According to a further aspect of the invention, there is provided a fusion protein comprising a recombinant protein as described herein, fused to a caseinomacropeptide.

In a further aspect, the invention provides a method of producing a recombinant protein, the method comprising culturing the host cell of the sixth aspect of the invention under conditions suitable for expression of the recombinant protein of the first aspect.

In a further aspect, the invention provides a recombinant protein obtained by the method of the seventh aspect.

In yet a further aspect, the invention provides a food composition obtained by the method of the seventh aspect.

In another aspect, the invention also provides a method of preparing a recombinant protein for the treatment of a subject with a proliferative disease, the method comprising:
- expressing in a host cell a recombinant protein which is devoid of at least one amino acid; and
- isolating the recombinant protein from the host cell.

Embodiments of the invention will now be described with reference to the accompanying figures in which:
Figure 1 is a schematic drawing of a recombinant fusion protein in accordance with embodiments of the invention;
Figure 2 is a schematic drawing of a recombinant protein, e.g. β-lactoglobulin (BLG), obtained from cleavage of the recombinant fusion protein of Figure 1;
Figure 3 shows a western blot of 6xHIS tagged BLG or BLG-linker-CMP, where the linker is the bovine chymosin recognition and cleavage sequence. Amino Acid substitutions are indicated with the eliminated amino acid (AA) within parentheses and the replacing AA immediately after the parentheses. _(C)VA indicates some C were replaced by V and some by A.
Figure 4 is a graph showing the titre presented as value relative to sample 1 (BLG native sequence) for 6xHIS tagged BLG or BLG-linker-CMP, where the linker is the bovine chymosin recognition and cleavage sequence.

The invention provides a recombinant protein comprising at least one amino acid substitution or deletion relative to a corresponding native protein. The term "corresponding native protein" refers to the naturally-occurring version of the same protein. The corresponding native protein has a wild-type amino acid sequence, whereas the recombinant protein has a modified amino acid sequence in which at least one amino acid present in the wild-type sequence has been deleted or substituted by another amino acid.

In some embodiments, each residue of a first type of amino acid that is present in the corresponding native protein has been independently deleted or substituted for another amino acid in the recombinant protein. By "independently deleted or substituted" it will be understood that all residues of the first type of amino acid may be deleted, all residues may be substituted (by the same or different amino acids), or some residues may be deleted and some may be substituted. For example, each serine residue that is present in the corresponding native protein may have been independently deleted or substituted for another amino acid in the recombinant protein, such that the recombinant protein is serine-free (i.e. devoid of serine). It may be that each serine residue is substituted for the same amino acid. Alternatively, the serine residues present in the native protein may be substituted by two or more different amino acids in the recombinant protein. In a further embodiment, one or more serine residues are deleted, while one or more other serine residues are substituted.

In some embodiments, each residue of a first type of amino acid that is present in the corresponding native protein has been independently deleted or substituted for another amino acid in the recombinant protein, and each residue of a second type of amino acid that is present in the corresponding native protein has been independently deleted or substituted for another amino acid in the recombinant protein. It may be that, in some embodiments, residues of a first, second, third or further type of amino acid are substituted in the recombinant protein. In other words, the recombinant protein may be devoid of two or more types of amino acids (e.g. 2, 3, 4 or 5 types). For example, the recombinant protein may be devoid of serine and glycine.

The invention thus provides recombinant proteins, and formulations comprising said proteins, which are tailor-made to exclude a specific amino acids or a set of amino acids while providing an adequate supply of other amino acids. In contrast to naturally occurring proteins, the amino acid sequence of a recombinant protein can be designed as required. Furthermore, the expression system used to produce the recombinant protein can be chosen and optimized to facilitate its isolation. Although designing a fully artificial protein from scratch is a viable and valid option, modifying the sequence of a known natural protein to match a specific amino acid composition can be an advantageous option. The modified protein can be expected to retain many of the structural and functional properties of the natural protein (e.g. stability, solubility, palatability, digestibility, non-toxicity). Advantageously, a recombinant protein which has been engineered to be free from (i.e. devoid of) one or more specific amino acids may provide a more convenient protein source for use in the treatment of patients with a proliferative disease who require a diet that is restricted in certain amino acids. Without being bound by theory, it is thought that the use of recombinant proteins in cancer starvation therapy may be more palatable for patients than a mixture of free amino acids, and/or may reduce or eliminate toxicity associated with the administration of free amino acids. Furthermore, since mammals have adapted to incorporate whole proteins into our diets, the use of recombinant proteins which are based on naturally occurring proteins as basis for a nutritional formulation is believed to reduce the disruptive effect of diet replacement based on free AAs.

Conveniently, the recombinant protein may be selected such that it is able to provide all of the amino acids required by the subject, with the exception of the specific amino acid(s) that are restricted. This enables the recombinant protein of the invention to be used as the sole source of protein which is consumed by the subject. Thus, in some embodiments, the recombinant protein comprises all nine essential amino acids. As is known in the art, essential amino acids are amino acids that cannot be synthesised by the body. The essential amino acids are histidine, isoleucine, leucine, lysine, methionine, phenylalanine, threonine, tryptophan and valine. However, it will be appreciated that a recombinant protein lacking an essential amino acid may be used therapeutically, for example for a short period of time.

Proteins which have relatively high levels of essential amino acids may be particularly suitable for use in the invention. In some embodiments, the recombinant protein comprises at least 10 wt%, at least 20 wt%, at least 30 wt%, at least 40 wt %, at least 50 wt%, at least 60 wt%, at least 70 wt%, at least 80 wt% or at least 90 wt% essential amino acids.

It will be appreciated that the amino acid(s) which the recombinant protein is deficient in will depend on the type of proliferative disease. Given that susceptibility to restriction of a particular AA or group of AAs depends on the specific genetic and metabolic makeup of the cell, different cancer types, or even different lines of a particular type, can be targeted with diets restricting different AAs. Guidance on a particular nutritional restriction treatment for a specific cancer type is provided by Table 4.

In some embodiments the recombinant protein is devoid of (i.e. free from) at least one amino acid selected from: alanine, arginine, asparagine, cysteine, glycine, glutamine, leucine, methionine, phenylalanine, proline, serine, threonine, tyrosine and valine.

In some embodiments, the recombinant protein is devoid of two or more specific amino acids. The recombinant protein may be devoid of proline and serine. The recombinant protein may be devoid of cysteine and methionine. The recombinant protein may be devoid of serine and glycine. The recombinant protein may be devoid of phenylalanine and tyrosine.

It will be appreciated that the amino acid(s) used to substitute those that are removed from the recombinant protein can be selected by the skilled person as required. For example, the amino acid(s) selected as replacements can be chosen either to increase the content of a particular AA in the modified protein, or to minimize the impact of the substitution on the modified protein's properties (e.g. structural stability, solubility, post-translational modifications). In the later case, guidance to assist with choice of substitute can be found in the literature, for example Betts, M.J. and Russell, R.B. Amino acid properties and consequences of substitutions, Bioinformatics for Geneticists, M.R. Barnes, I.C. Gray eds, Wiley, 2003.

It may be that the or each substitution present in the recombinant protein is a conservative substitution. As is known in the art, a conservative substitution is one wherein an amino acid is substituted by another amino acid having similar biochemical properties (e.g. charge, hydrophobicity, size and functional groups). Some example substitutions are presented in Table 1.

**Table 1**

| **AA substituted** | **Example substitutions** |
|---|---|
| Alanine | Serine, Glycine, Valine |
| Arginine | Lysine, Glutamine |
| Asparagine | Aspartate, Serine, |
| Aspartate | Glutamate, Asparagine |
| Cysteine | Alanine, Valine |
| Glutamate | Aspartate, Glutamine |
| Glutamine | Glutamate, Arginine |
| Glycine | Alanine, Asparagine, Serine |
| Histidine | Tyrosine, Asparagine |
| Isoleucine | Valine, Leucine |
| Leucine | Isoleucine, Methionine, Valine |
| Lysine | Arginine, Glutamate |
| Methionine | Leucine, Isoleucine |
| Phenylalanine | Tyrosine |
| Proline | Alanine |
| Serine | Asparagine, Threonine |
| Threonine | Serine |
| Tryptophan | Tyrosine, Phenylalanine |
| Tyrosine | Phenylalanine |
| Valine | Isoleucine, Leucine |

In some cases it may be preferable to eliminate a residue rather than to replace it.

In some embodiments:
(i) the first amino acid is arginine and each arginine residue present in the corresponding native protein has been independently replaced by lysine or glutamine;
(ii) the first amino acid is asparagine and each asparagine residue present in the corresponding native protein has been independently replaced by aspartate or serine;
(iii) the first amino acid is cysteine and each cysteine residue present in the corresponding native protein has been independently replaced by alanine or valine;
(iv) the first amino acid is glycine and each glycine residue present in the corresponding native protein has been independently replaced by alanine;
(v) the first amino acid is glutamine and each glutamine residue present in the corresponding native protein has been independently replaced by glutamate or arginine;
(vi) the first amino acid is leucine and each leucine residue present in the corresponding native protein has been independently replaced by isoleucine, methionine, or valine;
(vii) the first amino acid is methionine and each methionine residue present in the corresponding native protein has been independently replaced by isoleucine or leucine;
(vii) the first amino acid is phenylalanine and each phenylalanine residue present in the corresponding native protein has been independently replaced by tyrosine;
(vii) the first amino acid is proline and each proline residue present in the corresponding native protein has been independently replaced by alanine;
(viii) the first amino acid is serine and each proline residue present in the corresponding native protein has been independently replaced by asparagine or threonine;
(ix) the first amino acid is threonine and each threonine residue present in the corresponding native protein has been independently replaced by serine;
(x) the first amino acid is tyrosine and each tyrosine residue present in the corresponding native protein has been independently replaced by phenylalanine;
(xi) the first amino acid is valine and each valine residue present in the corresponding native protein has been independently replaced by isoleucine or leucine;
(xii) the first amino acid is glycine and each glycine residue present in the corresponding native protein has been independently replaced by threonine;
(xiii) the first amino acid is glycine and each glycine residue present in the corresponding native protein has been independently replaced by asparagine;
(xiv) the first amino acid is glycine and each glycine residue present in the corresponding native protein has been independently replaced by threonine, lysine or aspartate.

In some embodiments the recombinant protein has at least 80%, at least 85%, at least 90%, at least 95%, at least 96%, at least 97%, at least 98%, or at least 99% sequence identity to the corresponding native protein.

The term "identity", with reference to amino acid and nucleic acid sequences, is used herein to describe the level of similarity between two sequences. Sequence similarity or identity may be determined using standard techniques known in the art, including, but not limited to, the local sequence identity algorithm of Smith & Waterman, Adv. Appl. Math. 2, 482 (1981), by the sequence identity alignment algorithm of Needleman & Wunsch, J Mol. Biol. 48,443 (1970), by the search for similarity method of Pearson & Lipman, Proc. Natl. Acad. Sci. USA 85, 2444 (1988), by computerized implementations of these algorithms (GAP, BESTFIT, FASTA, and TFASTA in the Wisconsin Genetics Software Package, Genetics Computer Group, 575 Science Drive, Madison, Wl), the Best Fit sequence program described by Devereux et al., Nucl. Acid Res. 12, 387-395 (1984), or by inspection. Another suitable algorithm is the BLAST algorithm, described in Altschul et al., J Mol. Biol. 215, 403-410, (1990) and Karlin et al., Proc. Natl. Acad. Sci. USA 90, 5873-5787 (1993).

The skilled person will be capable of preparing a recombinant protein with a deletion or substitution of one or more specific amino acids, or one or more types of amino acids, and determining the effect of the deletion or substitution on the protein. For example, the skilled person is capable of experimentally testing the effect of a given substitution or deletion on expression levels, e.g. using the methods described herein. It may be that the level of production of the recombinant protein by a host cell, relative to the corresponding native protein, is indicative of the amino acid substitution or deletion resulting in a correctly folded protein.

In some embodiments, the recombinant protein is produced by a host cell in an amount which is at least 20%, at least 30%, at least 40%, at least 50%, at least 60%, at least 70%, at least 80%, at least 90%, at least 100%, at least 120%, at least 150%, or at least 200%, relative to the amount of the corresponding native protein produced when expressed under identical conditions (e.g. from the same expression cassette, in the same host, under the same environmental conditions).

A skilled person would also be able to directly assess the folding state and stability of a recombinant protein, and the effect of a given substitution or deletion on these parameters, using well established techniques such as circular dichroism spectroscopy (CDS), differential scanning calorimetry (DSC) or (for a version of the protein designed to remain intracellular) a cycloheximide chase assay. Detailed protocols for these techniques are readily available in the literature, e.g. Miles, A.J. et al. Tools and methods for circular dichroism spectroscopy of proteins: a tutorial review. Chem Soc Rev. 2021 50(15):8400-8413, Durowoju, I.B. et al. Differential Scanning Calorimetry - A Method for Assessing the Thermal Stability and Conformation of Protein Antigen. J Vis Exp. 2017 (121):55262, Miao, Y. et al. Cycloheximide (CHX) Chase Assay to Examine Protein Half-life. Bio Protoc. 2023 13(1 1):e4690.

The skilled person will also be able to determine other relevant properties of the native protein such as digestibility and allergenicity and determine the effect of a given substitution or deletion on this properties by applying methods readily available in the literature. For example: Sousa, R. et al. In vitro digestibility of dietary proteins and in vitro DIAAS analytical workflow based on the INFOGEST static protocol and its validation with in vivo data. Food Chem. 2023 404(B):134720, Tong, J.C. and Tammi, M.T. Methods and protocols for the assessment of protein allergenicity and cross-reactivity. Front Biosci 2008 13:4882-8, Tuppo, L. et al. Detection of Allergenic Proteins in Foodstuffs: Advantages of the Innovative Multiplex Allergen Microarray-Based Immunoassay Compared to Conventional Methods. Foods 2022 11(6): 878.

Additionally, or alternatively, the effect of a deletion or substitution on protein folding and/or expression may be predicted using a model, for example an artificial intelligence (Al) model such as AlphaFold2, RoseTTAFold or ESMFold.

In some embodiments, the recombinant protein is substantially unchanged in its stability, solubility, palatability, digestibility, and/or toxicity, relative to the corresponding native protein.

It is known in the art to classify proteins into the following categories: contractile, structural, storage, antibodies, enzymes, hormonal and transport.

In some embodiments, the recombinant protein is not an antibody, an enzyme or a hormonal protein.

In some embodiments, the protein is a contractile, structural, storage or transport protein.

Contractile proteins include actin, myosin and tropomyosin.

Structural proteins include actin, actinin, biglycan, collagen, decorin, fibrinogen, fibrin, elastin keratin, mucin, myelin associated glycoprotein, myosin, spectrin, tropomyosin, troponin, tubulin, vimentin and vitronectin.

Transport proteins include hemoglobin, cytochromes, myoglobin.

In some embodiments the recombinant protein is an animal protein (e.g. a milk protein or a meat protein) or a plant protein.

In some embodiments the animal protein is a meat protein. The meat protein may be selected from myosin, actin, myoglobin, collagen and elastin.

In some embodiments, the protein is a storage protein. These are proteins evolved to fulfil the nutritional requirements of organisms through different developmental stages and are the protein sources animals have adapted to and incorporated into their diets. The use of storage proteins as basis for a therapeutic recombinant protein is therefore expected to reduce the disruptive effect of diet replacement.

The storage protein may be an animal storage protein, or it may be a plant storage protein. It will be appreciated that the protein may be derived from any suitable species. Table 2 presents a non-exhaustive list of examples of storage proteins.

In some embodiments the animal storage protein is a milk protein. The milk protein may be casein, lactalbumin (e.g. alpha-lactalbumin) or lactoglobulin (e.g. beta lactoglobulin, BLG). The casein may be α-S1 casein, α-S2 casein, β-casein and kappa casein.

In some embodiments the animal storage protein is an egg protein. The egg protein may be selected from ovalbumin, ovaltransferrin, ovamucoid, flavoprotein, avidin, biotin, high-density lipoprotein (HDL), low-density lipoprotein (LDL), phosvitin, livetin, globulin and ovomucin.

In some embodiments, the plant storage protein is a protein derived from soy, wheat, maize, barley, rye, buckwheat, millet, oat, sorghum, pea, rice, cacao, oil seeds, cereals, legumes, nuts, lentil, chickpea, peanut, almond, coconut, algae, spirulina, quinoa, sunflower seed, canola seed, or hemp seed.

In some embodiments, the plant storage protein is selected from glycinin (e.g. glycinin G1, G2, G3, G4 or G5), beta-conglycinin (e.g. beta-conglycinin alpha 1, beta-conglycinin beta 1, beta-conglycinin alpha 2, or beta-conglycinin beta 2), alpha-gliadin, beta-gliadin, gamma-gliadin, avenin-like a1, avenin-like b1, B-1 hordein, B-3 hordein, C hordein, gamma hordein 1, Glutelin-2, 22 kDa alpha-zein 16, 22 kDa alpha-zein 14, alpha kafirin, avenin (e.g. avenin-3, avenin-E), vicilin, albumin (e.g. albumin-1a, albumin-2), or glutelin (e.g. glutelin type I or type-B 2).

**Table 2**

| **Protein** | **Type** | **Representative organism** | **Representative UniProt Accession #** |
|---|---|---|---|
| Beta Lactoglobulin (BLG) | Whey (milk) | Cow (Bos taurus) | P02754 |
| Alpha Lactalbumin (LALBA) | Whey (milk) | Cow (Bos taurus) | P00711 |
| Alpha S1 Casein | Casein (milk) | Cow (Bos taurus) | P02662 |
| Alpha S2 Casein | Casein (milk) | Cow (Bos taurus) | P02663 |
| Beta Casein | Casein (milk) | Cow (Bos taurus) | P02666 |
| Kappa Casein | Casein (milk) | Cow (Bos taurus) | P02668 |
| Ovalbumin | Albumin | Chicken (Gallus gallus) | P01012 |
| Glycinin G1 | Globulin (11s) | Soy (Glycine max) | P04776 |
| Glycinin G2 | Globulin (11s) | Soy (Glycine max) | P04405 |
| Glycinin G3 | Globulin (11s) | Soy (Glycine max) | P11828 |
| Glycinin G4 | Globulin (11s) | Soy (Glycine max) | P02858 |
| Glycinin G5 | Globulin (11s) | Soy (Glycine max) | P04347 |
| Beta-conglycinin alpha 1 | 7S seed storage family | Soy (Glycine max) | P11827 |
| Beta-conglycinin beta 1 | 7S seed storage family | Soy (Glycine max) | P25974 |
| Beta-conglycinin alpha 2 | 7S seed storage family | Soy (Glycine max) | P0DO15 |
| Beta-conglycinin beta 2 | 7S seed storage family | Soy (Glycine max) | F7J077 |
| Alpha/beta-gliadin | Prolamin | Wheat (Triticum aestivum) | P18573 |
| Gamma-gliadin | Prolamin | Wheat (Triticum aestivum) | P21292 |
| Avenin-like a1 | Prolamin | Wheat (Triticum aestivum) | Q2A784 |
| Avenin-like b1 | Prolamin | Wheat (Triticum aestivum) | Q2A783 |
| B-1 hordein | Prolamin | Barley (Hordeum vulgare) | P06470 |
| B-3 hordein | Prolamin | Barley (Hordeum vulgare) | P06471 |
| C hordein | Prolamin | Barley (Hordeum vulgare) | P06472 |
| Gamma hordein 1 | Prolamin | Barley (Hordeum vulgare) | P17990 |
| Glutelin-2 | Zein (Prolamin) | Maize (Zea mays) | P04706 |
| 22 kDa alpha-zein 16 | Zein (Prolamin) | Maize (Zea mays) | P04700 |
| 22 kDa alpha-zein 14 | Zein (Prolamin) | Maize (Zea mays) | P04698 |
| Alpha kafirin | Prolamin | Sorghum (Sorghum bicolor) | B1PHV3 |
| Avenin | Prolamin | Oat (Avena sativa) | P27919 |
| Avenin - 3 | Prolamin | Oat (Avena sativa) | P80356 |
| Avenin - E | Prolamin | Oat (Avena sativa) | Q09114 |
| Vicilin | 7S seed storage family | Pea (Pisum sativum) | P13918 |
| Albumin - 1a | Albumin | Pea (Pisum sativum) | P62926 |
| Albumin - 2 | Albumin | Pea (Pisum sativum) | P08688 |
| Vicilin | 7S seed storage family | Cacao (Theobroma cacao) | Q43358 |
| Glutelin type I | Globulin (11s) | Rice (Oryza sativa) | P07728 |
| Glutelin type-B 2 | Globulin (11s) | Rice (Oryza sativa) | Q02897 |

Preferably, the recombinant protein is selected from: casein, beta-lactoglobulin (BLG), ovalbumin, hordein, conglycinin and alpha-lactalbumin.

In some embodiments, the recombinant protein is beta-lactoglobulin (BLG).

In some embodiments the recombinant protein is a casein. The casein may be α-S1 casein, α-S2 casein and β-casein or κ-casein.

Certain embodiments of recombinant proteins and their amino acid substitutions are shown in Table 3.

**Table 3**

| **Recombinant protein** | **Substituted AA(s)** | **Replacement(s)** |
|---|---|---|
| BLG | Serine | Asparagine |
| BLG | Methionine | Leucine |
| BLG | Cysteine | Alanine and/or valine |
| BLG | Serine and glycine | Serine replaced by asparagine Glycine replaced by threonine |
| BLG | Serine and glycine | Serine replaced by asparagine Some glycine replaced by threonine, some by lysine and some by aspartate |
| BLG | Serine and glycine | Serine replaced by threonine Glycine replaced by asparagine |
| BLG | Arginine | Lysine |
| BLG | Serine and cysteine | Serine replaced by asparagine Some cysteine replaced by alanine and some by valine |
| BLG | Serine and arginine | Serine replaced by asparagine Arginine replaced by lysine |
| BLG | Serine, glycine and arginine | Serine replaced by asparagine Glycine replaced by threonine Arginine replaced by lysine |
| BLG | Serine, glycine and arginine | Serine replaced by asparagine Some glycine replaced by threonine, some by lysine and some by aspartate Arginine replaced by lysine |
| BLG | Serine, glycine and arginine | Serine replaced by threonine Glycine replaced by asparagine Arginine replaced by lysine |
| BLG | Cysteine and methionine | Some cysteine replaced by alanine and some by valine Methionine replaced by leucine |

Also disclosed are any of the embodiments shown in Table 3, provided (e.g. expressed) as a fusion protein comprising the recombinant protein fused to a caseinomacropeptide (CMP).

### composition

Also provided is a composition comprising a recombinant protein as described herein. Preferably, the composition is suitable for oral administration.

The composition may further comprise a pharmaceutically acceptable carrier. The recombinant protein may be dissolved, suspended or dispersed in the carrier. A "pharmaceutically acceptable carrier" refers to a carrier that, after administration to a subject, does not cause undesirable physiological effects. The carrier in a pharmaceutical composition must be "acceptable" also in the sense that is compatible with the recombinant protein. Suitable pharmaceutically acceptable carriers are well known in the art. For example, they may include, but not limited to, biocompatible vehicles (e.g. a liquid such as water, saline, glycerol or buffer solution, a gel or a solid matrix), adjuvants, additives (such as pH-adjusting additives), diluents, preservatives, or excipients such as fillers, binders, wetting agents, disintegrators, surface-active agents, lubricants and the like. The composition can be prepared by any method known in the art.

The composition may be formulated as a tablet, a capsule, a lozenge, a powder, a gel, a liquid or a solid.

In some embodiments, the composition is a food or beverage composition. The food or beverage composition may further comprise one or more components selected from:
(i) carbohydrates, e.g. one or more sugars such as glucose, fructose, galactose, lactose, sucrose, and/or one or more polysaccharides such as starch or cellulose;
(ii) fats, e.g., palm oil, rapeseed oil, soyabean oil, olive oil, avocado oil, nut oil, cocoa butter, pork lard, beef tallow, fish oils, omega-3 fatty acids, eicosapentaenoic acid, docosahexaenoic acid, alpha-linolenic acid, or oleic acid, or any combination thereof;
(iii) vitamins e.g., Vitamin B₁, Vitamin B₂, Vitamin B₃, Vitamin B₉, Vitamin B₁₂ Vitamin A, Vitamin C, Vitamin D, or Choline, or any combination thereof;
(iv) minerals (e.g.); iron, calcium, iodine, zinc, potassium, magnesium, selenium, sodium, copper, sulphur or cobalt, or any combination thereof, and/or;
(v) one or more further agents such as a flavorings, colours, sweeteners (e.g. polyols such as mannitol, sorbitol, xylitol, maltitol, lactitol, isomalt, erythritol, or polydextrose), pH modifiers, emulsifiers, preservative, and stabilizers.

Advantageously, formulating the composition as a food or beverage makes the recombinant protein more palatable for the subject, thereby improving compliance with treatment. Formulating the composition as a food or beverage also conveniently helps to ensure that the nutritional needs of the subject are met.

In some embodiments, the composition is devoid of proteins or polypeptides other than the recombinant protein according to the invention.

In some embodiments, the composition comprises a recombinant protein according to the invention which lacks a first amino acid, wherein the composition comprises a defined proportion of a further protein (e.g. a natural or recombinant protein) containing a predetermined amount of the first amino acid. No other proteins or polypeptides are present in the composition. In this way, the amount of the first amino acid received by a subject is controlled.

In one aspect, the invention provides a recombinant protein as described herein for use in the treatment of a proliferative disease or disorder in a subject, wherein the recombinant protein comprises at least one amino acid substitution relative to a corresponding native protein.

In some embodiments, the proliferative disease or disorder is a non-cancerous overgrowth or hyperproliferation of cells.

In some embodiments, the proliferative disease is cancer. In some embodiments the cancer is selected from: acute lymphoblastic leukemia, acute myeloid leukemia, adrenocortical carcinoma, AIDS-related cancers, AIDS-related lymphoma, anal cancer, appendix cancer, astrocytomas, basal cell carcinoma, bile duct cancer, bladder cancer, bone cancers, brain tumors, such as cerebellar astrocytoma, cerebral astrocytoma/malignant glioma, ependymoma, medulloblastoma, supratentorial primitive neuroectodermal tumors, visual pathway and hypothalamic glioma, breast cancer, bronchial adenomas, Burkitt lymphoma, carcinoma of unknown primary origin, central nervous system lymphoma, cerebellar astrocytoma, cervical cancer, childhood cancers, chronic lymphocytic leukemia, chronic myelogenous leukemia, chronic myeloproliferative disorders, colon cancer, cutaneous T-cell lymphoma, desmoplastic small round cell tumor, endometrial cancer, ependymoma, esophageal cancer, Ewing's sarcoma, germ cell tumors, gallbladder cancer, gastric cancer, gastrointestinal carcinoid tumor, gastrointestinal stromal tumor, gliomas, hairy cell leukemia, head and neck cancer, heart cancer, hepatocellular (liver) cancer, Hodgkin lymphoma, Hypopharyngeal cancer, intraocular melanoma, islet cell carcinoma, Kaposi sarcoma, kidney cancer, laryngeal cancer, lip and oral cavity cancer, liposarcoma, liver cancer, lung cancers, such as non-small cell and small cell lung cancer, lymphomas, leukemias, macroglobulinemia, malignant fibrous histiocytoma of bone/osteosarcoma, medulloblastoma, melanomas, mesothelioma, metastatic squamous neck cancer with occult primary, mouth cancer, multiple endocrine neoplasia syndrome, myelodysplastic syndromes, myeloid leukemia, nasal cavity and paranasal sinus cancer, nasopharyngeal carcinoma, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer, ovarian germ cell tumor, pancreatic cancer, pancreatic cancer islet cell, paranasal sinus and nasal cavity cancer, parathyroid cancer, penile cancer, pharyngeal cancer, pheochromocytoma, pineal astrocytoma, pineal germinoma, pituitary adenoma, pleuropulmonary blastoma, plasma cell neoplasia, primary central nervous system lymphoma, prostate cancer, rectal cancer, renal cell carcinoma, renal pelvis and ureter transitional cell cancer, retinoblastoma, rhabdomyosarcoma, salivary gland cancer, sarcomas, skin cancers, skin carcinoma merkel cell, small intestine cancer, soft tissue sarcoma, squamous cell carcinoma, stomach cancer, T-cell lymphoma, throat cancer, thymoma, thymic carcinoma, thyroid cancer, trophoblastic tumor (gestational), cancers of unknown primary site, urethral cancer, uterine sarcoma, vaginal cancer, vulvar cancer, Waldenstrom macroglobulinemia, and Wilms tumor.

In some embodiments the cancer is selected from: brain cancer (including glioblastoma, glioma and medullablastoma), bladder cancer (e.g. bladder melanoma), liver cancer (e.g. hepatocellular carcinoma), melanoma, colon cancer, colorectal cancer, prostate cancer, breast cancer, leukemia, ovarian cancer, kidney cancer, lung cancer (e.g. lung carcinoma), pancreatic cancer, uterine cancer, and head and neck cancer.

Table 4 presents a non-exhaustive list of examples of cancer types known to be susceptible to a particular AA restriction treatment.

**Table 4**

| **AA restricted in treatment** | **Cancer type** | **Cell line** | **Ref.** |
|---|---|---|---|
| Arginine | glioblastoma | ASS1 defective | Alexandrou et al. *Sci Rep* **2018** 8:12096 |
| | bladder | | As above |
| | hepatocellular carcinoma | | As above |
| | melanoma | | As above |
| | colon | HCT116 | As above |
| | colon | RKO | As above |
| | colorectal | OTC defective | As above |
| | colorectal | SW480 | As above |
| | hepatic | SNU-398 or Huh7 | Missiaen, R. et al. Cell Metab 2022 34(8):1151-1167 |
| | colon | CT26 | Satoh, Y. et al. Cancer Sci. 2020 111 (7):2248-2258 |
| | prostate | CWR22Rv1 | Hsu, S.C. et al. Theranostics 2021 11 (15):7527-7545 |
| Asparagine | breast | 4T1 | Knott. Nature 2018 554(7692):378-381 |
| | breast | SUM159PT | Krall, A.S. et al. Cell Metab 2021 33(5):1013-1026 |
| Cysteine | colon | HCT116 | Wu, J. et al. Signal Transduct Target Ther 2021 6(1): 188 |
| | | | Zhang, T. et al. Nat Metab 2020 2(10):1062-1076 |
| | colon | RKO | Wu, J. et al. Signal Transduct Target Ther 2021 6(1):188 |
| | colorectal | MC38 | Arensman, M.D. et al. Proc Natl Acad Sci USA 2019 116(19):9533-9542 |
| | glioma | IDH1-mutant NCH1681 | Ruiz-Rodado, V. et al. Mol Oncol 2022 16(9):1777-1794 |
| Glutamine | Medulloblastoma | | Niklison-Chirou, M.V. et al. Genes Dev 2017 31(17):1738-1753 |
| Leucine | breast | MCF-7 | Xiao, F. et al. Oncotarget 2016 7(39):63679-63689 |
| | melanoma | A-2058 | Sheen, J.H. et al. Cancer Cell 2011 19(5):613-28 |
| Methionine | breast | W-256 | Halpern, B.C. et al. Proc Natl Acad Sci USA 1974 71(4):1133-6 |
| | breast | MCF10AT1 | Hens, J.R. et al BMC Cancer 2016 16:349 |
| | leukemia | L1210 | Halpern, B.C. et al. Proc Natl Acad Sci USA 1974 71(4):1133-6 |
| | leukemia | J111 | Halpern, B.C. et al. Proc Natl Acad Sci USA 1974 71(4):1133-6 |
| | colon | CT26.WT cells | Jiménez-Alonso, J.J. et al. Int J Mol Sci 2023 24(5):4587 |
| | *AOM-induced colon carcinogenesis* | | Komninou, D. et al. Nutr Cancer 2006 54(2):202-8 |
| | colorectal | CRC119 and CRC240 | Gao, X. et al. Nature 2019 572(7769):397-401 |
| | ovarian | ID8 Trp53-/- | Jiménez-Alonso, J.J. et al. Int J Mol Sci 2023 24(5):4587 |
| | renal | Reca | Jiménez-Alonso, J.J. et al. Int J Mol Sci 2023 24(5):4587 |
| Proline | lung | A549, H358 | Liu, Y.et al. Oncogene 2020 39(11):2358-2376 |
| Serine | colon | PSAT1KO HCT116 | Montrose, D.C. et al. Cancer Res 2021 81 (9):2275-2288 |
| Threonine | breast | | Theuer, R.C. J Nutr 1971 101(2):223-32 |
| Valine | breast | BW10232 | Theuer, R.C. J Nutr 1971 101 (2):223-32 |
| | melanoma | | Sheen, J.H. et al. Cancer Cell 2011 19(5):613-28 |
| Cysteine and Methionine | colon | CT26 | Jiménez-Alonso, J.J. et al. Nutrients 2022 14(16):3378 |
| | glioma | KNS-42 | Upadhyayula, P.S. et al. Nat Commun 2023 14(1):1187 |
| | prostate | RP-B6 | Orillion, A. et al. Clin Cancer Res 2018 24(24):6383-6395 |
| Serine and Glycine | breast | 4T1 | Falcone, M. et al. Br J Cancer 2022 127(10): 1773-1786 |
| | colon | HCT116 | Maddocks, O.D.K. et al. Nature 2013 493(7433):542-546 |
| | | | Tajan, M. Nat Commun 2021 12(1):366 |
| | | | Maddocks, O.D.K. et al. Nature 2017 544(7650):372-376 |
| | | | Muthusamy, T. et al. Nature 2020 586(7831):790-795 |
| | colorectal | MC38 | Gravel, S.P. et al. Cancer Res 2014 74(24):7521-33 |
| | colorectal | DLD1 | Tajan, M. Nat Commun 2021 12(1):366 |
| Phenylalanine and Tyrosine | melanoma | S91 | Demopoulos, H.B. J Natl Cancer Inst 1966 37(2):185-90 |
| | melanoma | B16-BL6 | Elstad, C.A. et al Clin Exp Metastasis 1990 8(5):393-416 |
| | bladder melanoma | B16 | Abdallah, R.M. et al. J Natl Cancer Inst 1987 78(4):759-69 |
| | lung carcinoma | 3LL | Abdallah, R.M. et al. J Natl Cancer Inst 1987 78(4):759-69 |
| | hepatocarcinom a | RT7-4bs | Abdallah, R.M. et al. J Natl Cancer Inst 1987 78(4):759-69 |

In a series of embodiments:
(A) the cancer is brain cancer (e.g. glioblastoma, glioma, medulloblastoma), and the first amino acid(s) is (are): arginine; cysteine; glutamine; or cysteine and methionine;
(B) the cancer is bladder cancer, and the first amino acid(s) is (are): arginine; or phenylalanine and tyrosine;
(C) the cancer is liver cancer (e.g. hepatocellular carcinoma), and the first amino acid(s) is (are): arginine; or phenylalanine and tyrosine;
(D) the cancer is melanoma, and the first amino acid(s) is (are): arginine; leucine; valine; or phenylalanine and tyrosine;
(E) the cancer is colon or colorectal cancer and the first amino acid(s) is (are): arginine; cysteine; methionine; serine; cysteine and methionine; or serine and glycine;
(F) the cancer is prostate cancer and the first amino acid(s) is (are): arginine; or cysteine and methionine;
(G) the cancer is breast cancer and the first amino acid(s) is (are): asparagine; leucine; methionine; threonine; valine; or serine and glycine;
(H) the cancer is leukaemia and the first amino acid is methionine;
(I) the cancer is ovarian cancer and the first amino acid is methionine;
(J) the cancer is renal cancer and the first amino acid is methionine;
(K) the cancer is lung cancer and the first amino acid(s) is (are): proline; or phenylalanine and tyrosine.

As used herein, the term "treatment" refers to the decrease, prevention, delay, suppression, or elimination of any symptom of the proliferative disease (e.g. cancer). The treatment is not required to cure the disease but will provide a benefit in that the progression of the disease is delayed or prevented, one or more symptoms are ameliorated, the term of the disease is reduced, the subject's quality of life is improved and/or life span is extended. For example, the treatment may result in a reduction in the number and/or size of tumors, or in the prevention of tumor growth. In some embodiments, the treatment results in the prevention and/or reduction in metastases.

In some embodiments, the treatment results in a reduced rate of tumor growth or reduced metastases.

In some embodiments, the subject is maintained on a diet which is substantially deficient in the first amino acid (i.e. the amino acid which has been substituted in the recombinant protein). Thus, the first amino acid is eliminated from the subject's diet. By maintaining the subject on such a diet, the subject does not receive or consume any of the first amino acid through their diet, or by any other means

In some embodiments, the treatment comprises administering the recombinant protein in combination with an additional protein (natural or recombinant) which contains a predetermined amount of the first amino acid. It may be that the recombinant protein and the additional protein are the only sources of protein received by the subject during treatment. Rather than complete starvation of the first amino acid, this limits the amount of the first amino acid administered to the subject, which can have therapeutic value.

In some embodiments, the treatment further comprises surgery, radiotherapy, photodynamic therapy, cryotherapy, laser treatment, stem cell transplant and/or the administration of a further therapeutic agent. The further therapeutic agent may be a chemotherapeutic agent, a hormone, or an immunotherapeutic agent (e.g. an antibody or T-cell transfer therapy).

In some embodiments, the treatment further comprises administering a chemotherapeutic agent to the subject. The chemotherapeutic agent may be an alkylating agent (such as chlorambucil, cyclophosphamide, thiotepa, and busulfan, emozolomide, cisplatin, or mitomycin C), an antimetabolite (such as a purine antagonist, a pyrimidine antagonist, or a folate antagonist), an antitumor antibiotic (such as doxorubicin, mitoxantrone, or bleomycin), a genotoxic agent (such as cisplatin, gemcitabine, or topotecan), or a topoisomerase inhibitor (such as camptothecin or etoposide).

It will be appreciated that the amount of the recombinant protein that is administered to the subject will depend on a number of factors, including the age, weight and sex of the subject and any dietary restrictions.

In some embodiments, the recombinant protein may be the sole source of protein or amino acids for the subject. In other embodiments, the subject may receive one or more other sources of protein or amino acids.

In some embodiments, the recombinant protein may be administered to the subject in an amount of from 0.05 g to 2.5 g, from 0.1 g to 2.0 g, from 0.2 g to 1.5 g, from 0.3 g to 1.2 g, from 0.4 g to 1.0 g, or from 0.5 g to 0.8 g per kg per day.

In a further aspect, the invention provides a nucleic acid comprising a sequence encoding a recombinant protein as described herein. The nucleic acid molecule may be DNA or RNA. In some embodiments the nucleic acid molecule is DNA.

In some embodiments, the sequence encoding the recombinant protein is operably linked to a promoter. The skilled person will be aware of suitable promoters for expression of recombinant proteins in a given host. Preferred promoters may include those which are strong constitutive promoters in the presence of a particular carbon source (e.g. glucose, glycerol) or those which are strong regulated promoters (e.g. induced or derepressed under specific conditions such as presence or absence of a particular compound).

In some embodiments, the promoter is the GAP promoter (GAPp). The GAP promoter is the promoter from the Pichia pastoris TDH3 gene (Glyceraldehyde-3-phosphate dehydrogenase, GAPDH). In some embodiments the promoter is that of the *Pichia pastoris* GCW14 gene (GCW14p). In some embodiments the promoter is that of the *Pichia pastoris* AOX1 gene (AOX1p) or that of the Pichia pastoris HSP12 gene (HSP12p, also known a DHp).

In some embodiments, the sequence further comprises a terminator. Preferred terminators may include those which are associated with high levels of expression. Suitable terminators include those of the AOX1 and TDH3 genes of *Pichia pastoris,* and the terminators of the PGK1, CYC1, RPL3, and BNA4 genes of *Saccharomyces cerevisiae.* In some embodiments, the terminator is that of the Pichia pastoris AOX1 gene.

The promoter, the sequence which encodes the recombinant protein and the terminator (if present), which are comprised within the nucleic acid molecule, are together referred to as an "expression cassette". The invention thus also provides an expression cassette.

In some embodiments, the nucleic acid molecule further comprises one or more homology regions (e.g. two homology regions). These can be used to direct integration of the sequence encoding the recombinant protein, or the expression cassette, into the genome of a host cell. The skilled person will be capable of designing suitable homology regions using their common general knowledge. For example, the homology regions may be designed based on integration loci that have been successfully used previously, loci that are known to result in higher expression, and/or loci where integration does not disturb endogenous expression.

In some embodiments, the nucleic acid molecule, or the sequence encoding the recombinant protein, is codon-optimised for expression in a desired host cell, such as a bacterial cell or a eukaryotic cell (e.g. a yeast or a plant cell). In some embodiments the nucleic acid molecule, or the sequence encoding the recombinant protein, is codon-optimised for expression in *Pichia pastoris.*

The invention further provides a vector comprising the nucleic acid molecule described herein.

The vector may be a plasmid, a cosmid, or a viral vector.

In some embodiments, the vector is an expression vector for expression of the recombinant protein in a host cell. The expression vector may be a plasmid or a cosmid. It will be appreciated that an expression vector is a vector (e.g. a DNA vector) which can be introduced into a host cell and which will remain autonomous (i.e. it will replicate within the host cell and remain as an individual entity rather than be integrated in the genome) and from which proteins will be expressed.

In some embodiments, the vector is a cloning vector, such as a plasmid.

Also provided is a host cell comprising a nucleic acid molecule (e.g. an expression cassette) or a vector (e.g. an expression vector or a cloning vector) as described herein.

In some embodiments, the host cell comprises an expression cassette which is integrated into the genome of the host cell, wherein the expression cassette comprises a promoter, a sequence encoding the recombinant nucleic acid molecule operably linked to the promoter and, optionally, a terminator.

Integration of the expression cassette into the host cell genome can be carried out using standard techniques known to those skilled in the art, and as described herein. For example, the host cell may be co-transformed with: (i) a donor DNA molecule comprising the expression cassette located between two homology regions; and (ii) a plasmid comprising an RNA-guided endonuclease (CRISPR) system. The plasmid may further comprise an antibiotic resistance gene, enabling the selection of host cells which have been successfully transformed with the plasmid DNA. The proportion of plasmid and donor DNA in the transformation mix may be controlled so as to achieve a high probability that any host cell transformed with the plasmid is also transformed with the donor DNA molecule. The RNA-guided endonuclease (CRISPR) system ensures that the host cell genome has a double-strand cut at a target location, thereby promoting homology-driven recombination of the donor DNA molecule such that the expression cassette is integrated into the host cell genome.

In some embodiments, the host cell comprises multiple expression cassettes which are integrated into the host cell genome. In some embodiments, the host cell genome comprises two, three, four or more expression cassettes. All of the expression cassettes may be the same. For example, each expression cassette may comprise a sequence encoding the same recombinant protein. Insertion of multiple copies of the same gene (i.e. the sequence encoding the recombinant protein) can be useful to increase protein yield.

In embodiments wherein the host cell comprises multiple expression cassettes, each expression cassette may comprise a different promoter (and, optionally a different terminator). This may be advantageous to reduce the risk of unwanted recombinations.

In some embodiments, the host cell comprises an expression vector. The expression vector may comprise an expression cassette comprising a promoter, a sequence encoding the recombinant nucleic acid molecule operably linked to the promoter and, optionally, a terminator. The expression vector may be a plasmid. In some embodiments, the expression vector comprises multiple expression cassette, e.g. two, three, four or more expression cassettes.

The host cell may be a bacterial cell or a eukaryotic cell. A eukaryotic host can be a plant cell, an animal cell or a fungal cell. A fungal cell can be a yeast cell.

In some embodiments, the host cell is a eukaryotic cell selected from *Saccharomyces spp*., *Kluyveromyces spp*., *Pichia spp*., *Aspergillus spp*., *Tetrahymena spp., Yarrowla spp*., *Hansenula spp*., *Blastobotrys spp*., *Candida spp*., *Zygosaccharomyces spp*., *Debaryomyces spp*., *Fusarium spp*., and *Trichoderma spp.*

In some embodiments, the host cell is *Pichia spp*., such as *Pichia pastoris.*

In some embodiments, the host cell is a plant cell, such as soy.

A method of producing a recombinant protein comprises culturing a host cell as described herein under conditions suitable for expression of a recombinant protein as described herein.

Suitable conditions for culturing and expressing recombinant proteins in a given host cell will be known to those skilled in the art. For example, the expression of the recombinant protein in a eukaryotic host cell, such as *Pichia pastoris,* may be carried out under the conditions described herein.

In some embodiments, the method further comprises isolating the recombinant protein from the host cell culture. The method may further comprise purifying the recombinant protein. The recombinant protein may be separated and/or purified using one or more of the following techniques: centrifugation; precipitation (e.g. by adjusting the pH and/or salt composition of the culture medium); ultrafiltration; size exclusion chromatography; affinity chromatography; ion exchange chromatography; reverse phase chromatography; and immunoprecipitation.

While some proteins can be expressed recombinantly in good yield, it is known that other proteins are not expressed in high yield in a heterologous host. Well established methods to achieve high level titre and productivity of recombinant proteins in heterologous expression systems, such as expression under a strong promoter or insertion of multiple copies of the recombinant DNA, are not equally effective for all proteins. For example, α-S1 casein, α-S2 casein and β-casein do not respond well to these strategies. However, surprisingly the present inventors have found that the expression of a protein in a heterologous host can be significantly improved by fusing the casein, directly or indirectly, to a caseinomacropeptide (CMP), or a fragment thereof. Unexpectedly, the inventors have also found that by fusing the CMP to other proteins, even those (e.g. β-lactoglobulin) which are already expressed in heterologous hosts in good yield, the yield of those proteins can be increased.

Accordingly, in a further aspect the invention provides a recombinant protein as described herein, wherein the recombinant protein is fused to a caseinomacropeptide (CMP), or a fragment thereof.

The recombinant protein may be fused directly or indirectly to the CMP. By "fused" it will be understood that the recombinant protein and the caseinomacropeptide (CMP) or fragment thereof are joined directly or indirectly via peptide bonds such that they form two portions of a single, larger protein.

As used herein, the term "caseinomacropeptide" or "CMP" refers to the C-terminal portion of κ-casein (i.e. the portion which is released upon cleavage of κ-casein by chymosin, which may also be referred to as the "CMP domain") but excludes the chymosin recognition sequence. As is known in the art, bovine chymosin cuts κ-casein between an F residue and an M residue (residues 105 and 106 in bovine κ-casein) to release an N-terminal portion (para-kappa casein) and a C-terminal portion (comprising the caseinomacropeptide). The FM motif is contained within a recognition sequence, which in bovine κ-casein is HPHPHLSFMAIPPK (SEQ ID NO:1). Although, post-cleavage, a part of this recognition sequence resides in the C-terminal portion, this part of the recognition sequence is not considered to form a part of the CMP as defined in the context of this specification. However, in some embodiments the recombinant protein is joined to the CMP via a linker which may include some or all of the recognition sequence.

In some embodiments, the CMP comprises a sequence of no more than 150, no more than 120, no more than 100, no more than 90, no more than 80, no more than 75, no more than 72, no more than 70, no more than 65, no more than 64, no more than 60, or no more than 58 consecutive amino acids derived from the C-terminus of kappa casein. In other words, CMP may not comprise the full length kappa casein protein.

In some embodiments, the CMP comprises a sequence of 72 consecutive amino acids derived from the C-terminus of kappa casein. In some embodiments, the CMP comprises a sequence of at least 20, at least 25, at least 30, at least 35, at least 40, at least 45 or at least 50 consecutive amino acids derived from the C-terminus of kappa casein. In some embodiments, the CMP comprises a sequence of from 20 to 120, from 25 to 110, from 30 to 100, from 35 to 90, from 40 to 85, from 50 to 80, from 55 to 75, from 60 to 70 consecutive amino acids derived from the C-terminus of kappa casein.

The terms "cleavage site" and "recognition sequence" may be used interchangeably herein to refer to a sequence or chemical motif which is recognised by an enzyme or a chemical capable of cleaving the recombinant protein from the CMP.

The glycosylated form of CMP may also be referred to in the art as "GMP" or "gCMP" (glyco-caseino-macropeptide), while the non-glycosylated form is sometimes referred to as "aCMP" (aglyco-caseino-mactopeptide). It is also known in the art to use the term "CMP" for a heterogeneous mixture of non-glycosylated peptides and peptides with different degrees of glycosylation. In the context of the present specification, the term "CMP" is used to refer to both the non-glycosylated peptide and to glycosylated forms, unless otherwise stated.

In some embodiments, the fusion protein has increased hydrophilicity relative to the hydrophilicity of the recombinant protein alone. The CMP is a highly hydrophilic sequence, particularly in its glycosylated states. This is evident from the CMP sequence itself. Thus, in some embodiments the CMP is capable of conferring on the fusion protein an increased hydrophilicity, relative to the hydrophilicity of the recombinant protein alone. This may interfere with aggregation based on hydrophobic interactions and decrease the dependence of the protein's solubility on pH and on the concentration of other ions. This can result in more efficient biosynthesis, less intracellular degradation, better host-cell fitness and/or more efficient secretion.

In some embodiments the fusion protein has decreased hydrophobicity relative to the recombinant protein alone. Thus, the CMP may be capable of conferring on the fusion protein a reduced hydrophobicity, relative to the hydrophobicity of the (equivalent) recombinant protein alone.

The hydrophobicity of the fusion protein and/or the recombinant protein may be determined by Hydrophobic Partition, as described in Nakai S. 2003 Measurement of Protein Hydrophobicity; Current Protocols in Food Analytical Chemistry 9(1), Basic Protocol 5.

In some embodiments, the CMP is capable of increasing the solubility of the fusion protein in a medium, relative to the solubility of the (equivalent) recombinant protein alone in the same medium. Increased solubility may improve the expression of the recombinant protein in a heterologous system.

The effect of fusion to a CMP or fragment thereof on the solubility of a protein may be determined according to the following protocol:
1. Prepare multiple separate aliquots of fusion protein and recombinant protein alone at the same concentration (e.g. 2.7% w/w) in demineralised water adjusted to high pH with NaOH (e.g. pH 11);
2. Adjust the pH of each aliquot to a different (lower) value (e.g. decrease in 1 pH unit increments down to pH 1) with HCI, and incubate (e.g. 30 min with stirring) for equilibration;
3. Determine the resulting concentration in each condition by centrifugation of the equilibrated sample (e.g. 4000 g, 15 min) and of the original solution (pH 11), then remove the supernatant and add 1 volume of 2x SDS PAGE buffer to the supernatant. Run the samples on an SDS-PAGE gel and divide the intensity of the band obtained for each condition by the intensity of the band obtained at pH 11.

In some embodiments, the yield of the fusion protein may be greater than the yield of the (equivalent) recombinant protein alone when expressed under identical conditions (e.g. from the same expression cassette, in the same host, under the same environmental conditions).

In some embodiments, the CMP or fragment thereof comprises a sequence of at least 20, at least 25, at least 30, at least 35, at least 40, at least 45 or at least 50 consecutive amino acids.

In some embodiments, the CMP or fragment thereof comprises at least 3, at least 4, at least 5 glycosylation sites. In some embodiments, the CMP or fragment thereof comprises 6 glycosylation sites.

In some embodiments, the CMP comprises an N-terminal or a C-terminal truncation, relative to the full length CMP (as defined herein). In some embodiments, the CMP comprises both an N-terminal and a C-terminal truncation, relative to the full length CMP (as defined herein).

In some embodiments the CMP or fragment thereof comprises:
(i) the amino acid sequence:
   X3SGX2PX1 X1X1PTX3X2X3X3X2S X1X3X3X1X3X2X3X1 PX2 X3 (SEQ ID NO:2) wherein
   each X1 is independently selected from S and T,
   each X2 is independently selected from D and E, and
   each X3 is independently selected from A, I, L and V; or
(ii) an amino acid sequence which is at least 80%, at least 85%, at least 90% or at least 95% identical to SEQ ID NO: 2.

In some embodiments, the CMP or fragment thereof comprises an amino acid sequence which is at least 80%, at least 85%, at least 90% or at least 95% identical to SEQ ID NO: 2, with the proviso that at least 3, at least 4 or at least 5 of the X1 residues are retained. In some embodiments, all of the X1 residues are retained.

In some embodiments the CMP or fragment thereof comprises:
(i) an amino acid sequence selected from: 
X3SGX2PX1 X1X1PTX3X2X3X3X2S X1X3X3X1X3X2X3X1PX2 X3X3X2SPPX2X3NT
X3QX3X1STX3X3 (SEQ ID NO:3);
 and 
KNQX2KTX2X3PX1 X3NTX3X3SGX2PX1 X1X1PTX3X2X3X3X2S X1X3X3X1X3X2X3X1PX2
X3 (SEQ ID NO:4),
 or
(ii) an amino acid sequence which is at least 70%, at least 75%, at least 80%, at least 85%, at least 90% or at least 95% identical to SEQ ID NO: 3 or SEQ ID NO:4.

In some embodiments, the CMP or fragment thereof comprises an amino acid sequence which is at least 70%, at least 75%, at least 80%, at least 85%, at least 90% or at least 95% identical to SEQ ID NO: 3, with the proviso that at least 3, at least 4, at least 5 or at least 6 of the X1 residues are retained. In some embodiments, all of the X1 residues are retained.

In some embodiments, the CMP or fragment thereof comprises an amino acid sequence which is at least 70%, at least 75%, at least 80%, at least 85%, at least 90% or at least 95% identical to SEQ ID NO: 4, with the proviso that at least 3, at least 4, at least 5 or at least 6 of the X1 residues are retained. In some embodiments, all of the X1 residues are retained.

In some embodiments the CMP or fragment thereof comprises:
(i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:5);
 or
(ii) an amino acid sequence which is at least 70%, at least 75%, at least 80%, at least 85%, at least 90% or at least 95% identical to SEQ ID NO: 5.

In some embodiments, the CMP or fragment thereof comprises an amino acid sequence which is at least 70%, at least 75%, at least 80%, at least 85%, at least 90% or at least 95% identical to SEQ ID NO: 5, with the proviso that at least 3, at least 4, at least 5, at least 6 or at least 7 of the X1 residues are retained. In some embodiments, all of the X1 residues are retained.

In some embodiments the CMP or fragment thereof comprises:
(i) the amino acid sequence ASGEPT STPTIEAVES TVATLEASPE V (SEQ ID NO:6); or
(ii) an amino acid sequence which is at least 80%, at least 85%, at least 90% or at least 95% identical to SEQ ID NO: 6.

In some embodiments, the CMP or fragment thereof comprises an amino acid sequence which is at least 80%, at least 85%, at least 90% or at least 95% identical to SEQ ID NO: 6 with the proviso that at least 3, at least 4 or at least 5 glycosylation sites are retained (i.e. present). In some embodiments, 6 glycosylation sites are retained.

In some embodiments the CMP or fragment thereof comprises:
(i) an amino acid sequence selected from:
   ASGEPT STPTIEAVES TVATLEASPE VIESPPEINT VQVTSTAV (SEQ ID NO:7); and
   KNQDKTEIPT INTIASGEPT STPTIEAVES TVATLEASPE V (SEQ ID NO:8), or
   (ii) an amino acid sequence which is at least 70%, at least 75%, at least 80%, at least 85%, at least 90% or at least 95% identical to SEQ ID NO: 7 or SEQ ID NO:8.

In some embodiments, the CMP or fragment thereof comprises an amino acid sequence which is at least 70%, at least 75%, at least 80%, at least 85%, at least 90% or at least 95% identical to SEQ ID NO: 7, with the proviso that at least 3, at least 4, at least 5 or at least 6 glycosylation sites are retained (i.e. present). In some embodiments, 7 glycosylation sites are retained.

In some embodiments, the CMP or fragment thereof comprises an amino acid sequence which is at least 70%, at least 75%, at least 80%, at least 85%, at least 90% or at least 95% identical to SEQ ID NO:8 with the proviso that at least 3, at least 4, at least 5 or at least 6 glycosylation sites are retained (i.e. present). In some embodiments, 7 glycosylation sites are retained.

In some embodiments the CMP or fragment thereof comprises or consists of:
(i) the amino acid sequence
   KNQDKTEIPT INTIASGEPT STPTIEAVES TVATLEASPE VIESPPEINT VQVTSTAV (SEQ ID NO:9); or
(ii) an amino acid sequence which is at least 70%, at least 75%, at least 80%, at least 85%, at least 90%, at least 92%, at least 95%, at least 96% or at least 98% identical to SEQ ID NO: 9.

In some embodiments the CMP or fragment thereof comprises (an amino acid sequence which is at least 70%, at least 75%, at least 80%, at least 85%, at least 90%, at least 92%, at least 95%, at least 96% or at least 98% identical to SEQ ID NO: 9, with the proviso that at least 3, at least 4, at least 5, at least 6 or at least 7 glycosylation sites are retained (i.e. present). In some embodiments, 8 glycosylation sites are retained.

In some embodiments, the CMP comprises or is constituted by an amino acid sequence which is at least 70%, at least 75%, at least 80%, at least 85%, at least 90%, at least 95%, at least 96%, at least 97%, at least 98%, or at least 99% identical to the CMP of cow (*Bos taurus*) κ-casein (Uniprot accession no. P02668). The cow κ-casein may be variant B, or it may be the reference variant A or any one of the other variants (B2, E, G, or F).

The amino acid sequence of the CMP from cow κ-casein protein (variant B) is designated herein as SEQ ID NO:9.

The CMP, or fragment thereof, may be derived from any suitable mammal. In some embodiments, the CMP comprises or is constituted by an amino acid sequence which is at least 70%, at least 75%, at least 80%, at least 85%, at least 90%, at least 95%, at least 96%, at least 97%, at least 98%, or at least 99% identical to the wild-type CMP of κ-casein from goat, sheep, camel, donkey, horse, human, rat, mouse, guinea pig, pig, water buffalo, rabbit, whale (e.g. fin whale, beluga whale), giraffe or elephant.

In any of the embodiments of the CMP or fragment thereof described herein, each residue of the first amino acid that is present in a corresponding native CMP may be substituted for another amino acid. Accordingly, this results in the CMP or fragment thereof being devoid of the first amino acid, in addition to the recombinant protein being devoid of that amino acid. Thus, in some embodiments the entire fusion protein is devoid of the first amino acid. This conveniently enables the fusion protein (i.e. the recombinant protein - CMP fusion) to be administered to a subject without first having to cleave the recombinant protein from the CMP or fragment thereof.

In some embodiments each serine residue present in the corresponding native CMP or fragment thereof is substituted by another amino acid, optionally wherein each serine residue is substituted by threonine. Additionally or alternatively, each glycine residue present in the corresponding native CMP or fragment thereof may be substituted by another amino acid, optionally wherein each glycine residue is substituted by alanine.

Thus, in some embodiments the CMP or fragment thereof comprises or consists of an amino acid sequence selected from: SEQ ID NO: 2; SEQ ID NO:3; SEQ ID NO:4; EQ ID NO:5; SEQ ID NO:6; SEQ ID NO:7; SEQ ID NO:8 or SEQ ID NO:9, wherein each serine residue has been substituted (i.e. replaced) by threonine and/or wherein each glycine residue has been substituted by alanine.

In some embodiments, the fusion protein comprises a linker between the recombinant protein and the CMP, or a fragment thereof.

The linker may comprise an amino acid sequence of from 3 to 50, from 5 to 40, from 8 to 30, from 10 to 20 or from 12 to 15 residues in length.

In some embodiments, the linker comprises a cleavage site. Advantageously, the cleavage site can be used to cleave the fusion protein so as to separate the recombinant protein from the peptide. The cleavage site may be a chemical cleavage site or an enzyme cleavage site.

In some embodiments, the cleavage site is an enzyme cleavage site. The enzyme cleavage site may be a hydrolase cleavage site (such as a protease cleavage site) or a peptide lyase cleavage site (such as an amidine lyase cleavage site). In some embodiments the cleavage site is a protease cleavage site. The cleavage site may be a chymosin cleavage site, or an enteropeptidase cleavage site. A suitable enteropeptidase cleavage site may be that of bovine enteropeptidase (Uniprot accession number P98072).

In some embodiments, the cleavage site is a chymosin cleavage site. The chymosin cleavage site may be that of any mammal. For example, the chymosin cleavage site may be bovine, goat, sheep, or camel. In some embodiments, the cleavage site is derived from the same species as the CMP. In some embodiments the chymosin cleavage site comprises the amino acid sequence HLSFXAIP (SEQ ID NO.10), wherein X is L, M or F. Cleavage occurs between the F and X residues. Some additional residues are believed to make the interaction with the enzyme more stable. Therefore, in some embodiments the chymosin cleavage site comprises the sequence HPHPHLSFMAIPPK (SEQ ID NO.1).

In some embodiments, the chymosin cleavage site comprises the sequence FLQKQQYGISSKFX wherein X is selected from: W, Y, F, C, R, I, M, H, L, V, K, N, T or A(SEQ ID NO.11). Preferably, X is W, Y or F. In some embodiments, the chymosin cleavage site comprises the sequence RPRPRPSFIAIPPK (SEQ ID NO.12). Other suitable cleavage sites which may be incorporated into the fusion protein will be known to those skilled in the art, such as those described in WO2022/072718.

The linker or the cleavage site may be devoid of the first amino acid. Thus, in any embodiments of the linker or cleavage site described herein, residues of the first amino acid may be substituted by another amino acid. In some embodiments, any serine residues present in the linker or cleavage site may be substituted by threonine. Additionally or alternatively, any methionine residues present in the linker or cleavage site may be substituted by leucine.

For example, in some embodiments the linker comprises an amino acid sequence according to SEQ ID NO:1, SEQ ID NO:10 or SEQ ID NO:12 in which the serine residue is replaced by threonine. In some embodiments the linker comprises an amino acid sequence according to SEQ ID NO:1 in which the methionine residue is replaced by leucine.

The recombinant protein may be located at or towards the N-terminal end of the fusion protein, and the CMP may be located at or towards the C-terminal end of the fusion protein. Alternatively, the recombinant protein may be located at or towards the C-terminal end of the fusion protein, and the CMP may be located at or towards the N-terminal end of the fusion protein.

As noted above, it has been found that excess or undesirable proliferation of cells can be reduced or prevented by depriving the cells of one or more particular amino acids. The specific amino acid(s) that the cells can be deprived of in order to prevent or reduce undesirable proliferation may depend on the type of proliferative disease. Accordingly, the specific amino acids that are required to be replaced or deleted in the recombinant protein of the invention, relative to the corresponding native protein, may depend on the type of disease that the subject is suffering from. Advantageously the use of a recombinant protein enables the specific amino acid substitutions or deletions to be tailored according to the subject's diagnosis.

Thus in a further aspect, the invention provides a method of providing a recombinant protein for the treatment of a subject with a proliferative disease, the method comprising:
- expressing in a host cell a recombinant protein which is devoid of at least one amino acid; and
- isolating the recombinant protein from the host cell.

The method may further comprise purifying the isolated recombinant protein.

The step of expressing the recombinant protein in the host cell may comprise culturing the host cell under conditions suitable for expression of the recombinant protein.

The method may further comprise identifying an amino acid which modulates (e.g. increases) the proliferation and/or the survival of cells associated with the proliferative disease (e.g. cancer or tumour cells). It may be that in the presence of the amino acid, the cells have an increased proliferation rate and/or survival rate, and that in the absence of the amino acid, the cells have a decreased proliferation rate and/or survival rate.

The amino acid may be identified using a diagnosis received by the subject (e.g. the type of cancer which the subject is suffering from). For example, a skilled person may be capable of determining which amino acid(s) to delete or substitute for a specific type of cancer using their common general knowledge and/or the available literature, for example that listed in Table 4.

In many cases the susceptibility of a given cancer type to a particular AA restriction treatment is not only known as an empirical correlation, but is instead traced to specific genetic and metabolic deficiencies. This is of particular importance since such deficiencies constitute markers that can be scanned relatively quickly and serve to predict the susceptibilities of an individual case. For example, cellular production of the AA arginine uses argininosuccinate as a substrate. Argininosuccinate is produced by the condensation of citrulline and aspartate, a reaction catalysed by the enzyme ASS1, while citrulline is produced by conjugation of ornithine and carmaboylphosphate, mediated by the enzyme OTC. Therefore, low or absent expression of the ASS1 or OTC genes can be used as a marker to predict dependency on dietary arginine.

Thus, in some embodiments the method comprises determining the dependency of the proliferative disease (e.g. the cancer) on one or more amino acids. In some embodiments, the method comprises determining which amino acid(s) contribute to, or are essential for, the unwanted proliferation of cells associated with the disease or disorder (e.g. cancerous cells).

In some embodiments, identifying an amino acid which modulates the proliferation and/or survival of cells associated with the disease (e.g. cancerous cells or tumour cells) is carried out using metabolomic and/or genomic analysis. Methods of identifying the nutrient-dependency (including the amino-acid dependency) of a cancer are described in WO2023130140, the contents of which are incorporated herein by reference.

In some embodiments, identifying the amino acid which modulates the proliferation and/or survival of cells associated with the disease (e.g. cancerous cells or tumour cells) is carried out using a machine learning model. An example of a system which implements a machine learning model which is configured to identify metabolic targets is described in WO2023044475, the contents of which are incorporated herein by reference.

The method may further comprise preparing a nucleic acid molecule comprising a sequence encoding the recombinant protein. The method may further comprise introducing the nucleic acid molecule into the host cell.

### Example 1: Expression of recombinant proteins with native and modified AA profiles

A library of constructs for expression of following recombinant proteins was prepared:
(1) Wild-type BLG
(2) Wild-type BLG-CMP fusion
(3) BLG with serine to asparagine substitution
(4) BLG-CMP fusion wherein serine residues were substituted by asparagine in BLG and by threonine in CMP
(5) BLG with methionine to leucine substitution
(6) BLG-CMP fusion wherein methionine residues were substituted by leucine in BLG
(7) BLG with cysteine to alanine/valine substitution
(8) BLG-CMP fusion wherein cysteine residues were substituted by alanine and valine in BLG

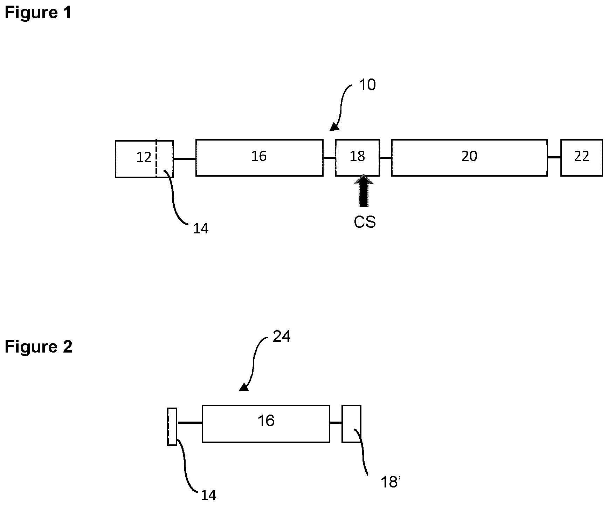

With reference to Figure 1, the constructs encoding a BLG-CMP fusion were designed to include a DNA sequence encoding a recombinant fusion protein 10 comprising, from the C-terminus to the N-terminus: a MF-α57-70 signal sequence from *S*. *cerevisiae* 12; a sequence encoding BLG (wild-type of substituted version) 16; a linker 18 comprising a cleavage site (CS); a caseinomacropeptide 20; and a HIS tag 22. The DNA sequence was operably linked to promoter and terminator sequences. Homology regions to direct chromosomal integration are also fused to these DNA molecules. The resulting DNA constructs were separately transformed into *Pichia pastoris* host cells, which were cultured.

It will be appreciated that in a recombinant fusion protein 24 secreted by the host cell, the signal sequence will have be cleaved (indicated by the dotted line), potentially leaving behind a short fragment of the signal sequence 14 in the expressed protein. While the fusion protein shown in the embodiment of Figure 1 comprises a HIS tag, it will be appreciated that in other embodiments the recombinant fusion protein may contain a different tag, multiple tags or no tags.

### Methodology

### Preparation of constructs

1. Protein and DNA sequences of bovine β-Lactoglobulin and kappa casein were obtained from databases. The codon usage of protein coding DNA was optimized for expression in *Pichia pastoris.*
2. DNA sequences coding for a promoter (GCW14p: promoter of the *Pichia pastoris GCW14* gene), a signal sequence (SS - *S*. *cerevisiae* α-mating factor Δ57-70 - MF-α57-70), either BLG or kappa casein (CSN3), a tag [GSGS-6xHIS (SEQ ID NO: 21)] and terminator (AOX1t: terminator of the *Pichia pastoris* AOX1 gene) were produced by *in vitro* DNA synthesis and onboarded in a cloning vector (pUC19), resulting in the circular vectors
   a) pUC19-Promoter-SS-BLG-Tag-Terminator
   b) pUC19-Promoter-SS-CSN3-Tag-Terminator
3. A ca. 1000 bp region upstream of the genomic loci targeted for insertion was amplified from genomic DNA by PCR using a standard PCR protocol, thereby creating a first homology region (HRL).
4. Aca. 1000 bp region downstream of the genomic loci targeted for insertion was amplified from genomic DNA by PCR using a standard PCR protocol, thereby creating a second homology region (HRR).
   The primers used in steps 3 and 4 to create the homology regions were designed with overhangs to allow assembly of the resulting fragments and a linearized cloning vector through a standard Gibson Assembly protocol (i.e. the reverse primer for HRL has an overhang that overlaps the forward primer for HRR, the forward primer for HRL has an overhang that overlaps the reverse primer from step 5 and the reverse primer for HRR has an overhang that overlaps the forward primer from step 5).
5. A cloning vector (pUC19) was linearized by PCR with primers that amplify the entire vector except the MCS.
6. The DNA fragments resulting from steps 3, 4 and 5 where assembled to form a circular vector by a standard Gibson Assembly protocol
   c) pUC19-HRL-HRR
7. A DNA fragment encoding the expression cassette Promoter-SS-BLG-Tag-Terminator was amplified by PCR from vector (a) of step 2.
8. The vector (c) of step 6 was linearized by PCR with primers containing overhangs designed to overlap the first bases of the promoter and last bases of the terminator from step 7 (i.e. a reverse primer binding the 3' end of HRL with an overhang complementary to the 5' end of the promoter and a forward primer binding the 5' end of HRR with an overhang complementary to the 3' end of the terminator).
9. The linearized vector from step 8 was assembled by a standard Gibson Assembly protocol into a circular vector with the DNA fragment from step 7, resulting in the circular vector:
   d) pUC19-HRL-Promoter-SS-BLG-Tag-Terminator-HRR
10. A DNA fragment encoding the 5' portion of the expression cassette Promoter-SS-BLG (i.e. excluding the tag and terminator) was amplified by PCR from the vector (a) of step 2.
11. A DNA fragment encoding the 3' portion of the expression cassette linker-CMP-tag-terminator (i.e. excluding promoter-SS-para-K-casein) was amplified by PCR from vector (b) of step 2. Where K-casein is para-K-casein-linker-CMP and the linker is the recognition and cleavage sequence of chymosin. The forward primer contained an overhang complementary to the 3' end of BLG.
12. The DNA fragments from steps 8, 10 and 11 where assembled into a circular vector by a standard Gibson Assembly protocol resulting in the circular vector
   e) pUC19-HRL-Promoter-SS-BLG-linker-CMP-Tag-Terminator-HRR where the linker is the recognition sequence and cleavage site of bovine chymosin.
13. The codons coding for the amino acids Serine (S) and Methionine (M) where individually replaced in the sequence of BLG by codons corresponding to Asparagine (N) and Leucine (L), respectively. Generating the sequences BLG_(S)N and BLG_(M)L. The codons coding for cysteine where replaced either by codons coding for alanine (A) or coding for valine (V), generating the sequence BLG_(C)VA. These sequences were produced by *in vitro* DNA synthesis with a 5' extension corresponding to the last 24 base pairs on the 3' end of the SS MF-α57-70 and a 3' extension coding for a tag (GGG-6xHIS) followed by 26 base pairs corresponding to the 5' end of the AOX1 terminator.
14. The vector (d) of step 9 was linearized by PCR with a reverse primer on the 3' end of the SS MF-α57-70 and a forward primer on the 5' end of the terminator (i.e. excluding BLG-Tag).
15. The linearized vector of step 14 was assembled by a standard Gibson Assembly protocol with the synthetic DNA fragments of step 13 (individually), generating the circular vectors
   f) pUC19-HRL-Promoter-SS-BLG_(S)N-Tag-Terminator-HRR
   g) pUC19-HRL-Promoter-SS-BLG_(M)L-Tag-Terminator-HRR
   h) pUC19-HRL-Promoter-SS-BLG_(C)VA-Tag-Terminator-HRR
16. A DNA fragment corresponding to the 3' end of bovine kappa casein (linker-CMP where the linker is the recognition and cleavage sequence of bovine chymosin) was produced by *in vitro* DNA synthesis substituting all codons coding for serine by codons coding for threonine: CMP_(S)T. The DNA fragment was produced with a 5' extension corresponding to the last 6 base pairs (3') of BLG and a 3' extension coding for a tag (GGG6xHIS) followed by a stop codon, 6 more base pairs and the first 42 base pairs (5') of the AOX1 terminator.
17. the vector (f) of step 15 was linearized by PCR with a reverse primer annealing on the 3' end of BLG (i.e. excluding the tag) and a forward primer annealing on the 5' end of the terminator. The reverse primer contained an overhang (18 base pairs) complementary to the 5'end of the synthetic DNA fragment form step 16.
18. The DNA fragments from steps 16 and 17 where assembled by a standard Gibson Assembly protocol into the circular vector
   i) pUC19-HRL-Promoter-SS-BLG_(S)N-linker-CMP_(S)T-Tag-Terminator-HRR
19. A DNA fragment coding for linker-CMP-tag-terminator, were the linker is the recognition and cleavage sequence of bovine chymosin, was amplified by PCR form the vector (e) of step 12.
20. The vectors (g) and (h) were linearized by a standard PCR reaction with reverse primers annealing on the 3' end of BLG_(M)L or BLG_(C)VA and a forward primer annealing on the 5'end of the terminator (i.e. excluding the tag). The reverse primers had overhangs complimentary to the 5' end of the DNA fragment from step 19
21. The DNA fragment from step 19 was assembled with the linearized vectors of step 20 (individually) by a standard Gibson assembly protocol generating the circular vectors
   j) pUC19-HRL-Promoter-SS-BLG_(M)L-linker-CMP-Tag-Terminator-HRR
   k) pUC19-HRL-Promoter-SS-BLG_(C)VA-linker-CMP-Tag-Terminator-HRR
   Note that the linker-CMP is naturally free of cysteine. The CMP is also free of methionine, however this linker has one M residue. Therefore, the vector (g) codes for a methionine free protein, while the vector (j) codes for a methionine poor protein. Further versions of BLG_(M)L_CMP fully devoid of methionine can be produced by equivalent methods using a different linker.
22. Donor DNA was prepared from the vectors (d), (e), (f), (g), (h), (l), (j) and (k) of steps 9, 12, 15, 18 and 21 by a standard PCR reaction with a forward primer annealing at the 5' end of HRL and a reverse primer annealing at the 3' end of HRR followed by agarose gel electrophoresis and extraction of the pertinent DNA band from the gel.

At all pertinent steps, correct amplification and assembly of DNA was verified by restriction analysis and/or sequencing.

### Transformation of host cells

23. Electro-competent *Pichia pastoris* cells (strain NRRL Y-11430 also known as CBS7435 or ATCC 76273) were prepared from exponentially growing cells by treatment with lithium acetate and sorbitol as described by [54].
24. The electro-competent cells of step 23 were mixed with the donor DNA molecules of step 22 (individually) and with an episomal vector expressing an RNA guided endonuclease, guide RNA specific for the genomic loci targeted for insertion and an antibiotic resistance gene.
25. The mix of step 24 was transferred to an electroporation cuvette, subjected to a pulse (2 kV, 25 µF, 200 ohm), diluted with YPD growth media (Yeast extract 1%, Peptone 2%, Dextrose 2%), transferred to a fresh vial, allowed to recover for 2 hours at 30°C and spread on a YPD-Agar (2%) plate with an antibiotic for selection according to the resistance provided by the vector of step 24.
26. After 2-3 days of growth at 30°C single transformed colonies were picked from the selective YPD-Agar plate of step 25 and analysed by a standard colony PCR protocol with primers specific for the targeted genomic locus and the donor DNA.
27. Clones identified as positive (i.e. with a correctly integrated expression cassette) in step 26 were spread on non-selective YPD-Agar plates.
28. After 2 days at 30°C single colonies were picked from the plates of step 27 and spread on selective and non-selective plates. This step was repeated, passing cells from the non-selective plates onto selective and non-selective plates, until there was no growth on selective plates (indicating loss of the episomal vector).

### Host cell culture

29. Cells from step 28 were inoculated in 3 ml YPD and grown at 28 °C for ca. 16 h, cultures were then diluted 1:10 in YPD and grown until mid-exponential phase (ca. 5 h) at 28 °C.
30. The pre-cultures of step 29 were diluted to OD600 0.2 in 30 ml of BMDY media (2% peptone, 1% yeast extract, 100 mM potassium phosphate pH 6, 13.4 g/L YNB with ammonium sulphate, 2 % glucose 0.4 mg/L biotin) and grown for 22 h at 25 °C (ca. OD600 20).
31. Samples from the cultures of step 30 where collected by removing 4 ml from the culture.

### Protein recovery

32. Secreted proteins in the samples of step 31 were separated from the cells by centrifugation, 5 min 1500 rcf.

### Protein Visualisation and Determination of expression levels.

### SDS-PAGE - WB

33. An aliquot (e.g. 10 ul) of a 1:4 dilution of the supernatant from step 32 was mixed with (e.g. 2 ul) 6X Laemmli sample buffer with 0.6 M dithiothreitol (DTT) and incubated at 65°C for 10 minutes.
34. The samples from step 33 were separated through a standard SDS-PAGE electrophoresis protocol and analysed through a standard western blot protocol with an antibody specific for the tags (HIS)

### ELISA

35. Recombinant BLG or BLG-CMP fusion in a fraction of the supernatants from step 32 (e.g. 200 µl) were diluted as required (e.g. 500x) in PBS and quantified by a standard ELISA protocol using an HRP conjugated antibody specific against the tag (HIS), an HRP substrate (ABTS) and a calibration curve with a tagged protein of known concentration (HIS tagged FKBP12). To calculate protein concentration from detected absorbance, the differences in molecular weight of the standard and the different recombinant proteins were taken into account.

### Results

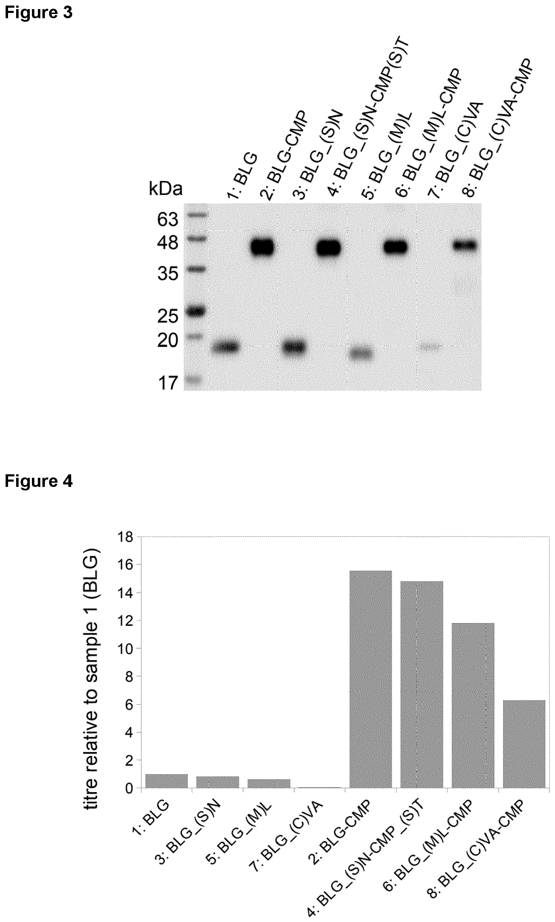

With reference to Figure 3, wild-type recombinant BLG is seen on a western blot as a single band (1) at the expected size (under 20 kDa). The fusion BLG-CMP is also seen as a single band (2). Due to glycosylation of the CMP the fusion protein appears at a higher molecular weight (above 35 kDa) than the 26 kDa predicted by the amino acid sequence alone.

BLG with either serine (3) or methionine (5) residues substituted produce bands of comparable intensity to BLG with native sequence (1). In contrast BLG with cysteine residues substituted (7) is barely detectable.

Bands of BLG-CMP are much more intense than bands of BLG, either with native sequence (2) or with the serine, methionine or cystine substitutions (4, 6, 8), indicating that the fusion protein is produced with higher titre. This effect is particularly dramatic for the protein with cysteine substitutions (8).

The results of the ELISA corroborate this (Figure 4). BLG with serine (3) or methionine (5) substituted are produced with a titre comparable to BLG with native sequence (1) (80 and 60% respectively). In contrast the titre of BLG without cysteines (7) is only 5% that of the unmodified protein, suggesting that loss of cysteine bridges has a strong destabilizing effect. The BLG-CMP fusion proteins (wt, S and M substitutions (2, 4, 6)) are produced with a titre between 15- and 12-fold that of the non-fusion protein with native sequence. Although the titre of the fusion protein BLG_(C)VA-CMP (8) is only 40% that of the fusion protein with unmodified sequence (2), this represents a 125 fold increase over the non fusion BLG_(C)VA protein (7).

### Sequences used in Example 2

Secreted BLG-linker-CMP where the linker (underlined) is the bovine chymosin recognition and cleavage sequence:

EAEALIVTQTMKGLDIQKVAGTWYSLAMAASDISLLDAQSAPLRVYVEELKPTPEGDLEILLQKWE
NGECAQKKIIAEKTKIPAVFKIDALNENKVLVLDTDYKKYLLFCMENSAEPEQSLACQCLVRTPEV
DDEALEKFDKALKALPMHIRLSFNPTQLEEQCHIHPHPHLSFMAIPPKKNQDKTEIPTINTIASGEP
TSTPTIEAVESTVATLEASPEVIESPPEINTVQVTSTAVGSGSHHHHHH (SEQ ID NO:13).

DNA sequence coding for a protein according to SEQ ID NO:13. Secretion signal, linker and tag underlined. Codon optimized for expression in *Pichia pastoris.*

ATGAGATTTCCATCTATTTTCACTGCTGTTTTGTTTGCTGCTTCTTCTGCTTTGGCTGCTCCAG
TTAACACTACTACTGAAGATGAGACTGCTCAAATTCCTGCTGAAGCTGTTATTGGTTACTCTGA

TTTGGAGGGAGATTTTGATGTTGCTGTTTTGCCTTTCTCTGCTTCTATCGCTGCTAAGGAAGA
GGGTGTTTCTTTGGAAAAGAGAGAAGCTGAGGCTTTGATTGTTACTCAAACTATGAAGGGTT
TGGATATCCAAAAGGTTGCTGGTACTTGGTACTCTTTGGCTATGGCTGCTTCTGATATTTCTTT
GTTGGATGCTCAATCTGCTCCATTGAGAGTTTATGTTGAAGAGTTGAAACCAACTCCTGAAGG
AGATTTGGAAATTTTGTTGCAAAAGTGGGAAAACGGAGAGTGTGCTCAAAAGAAAATTATCGC
TGAAAAGACTAAGATCCCTGCTGTTTTTAAAATCGATGCTTTGAACGAGAATAAGGTTTTGGTT
TTGGATACTGATTACAAGAAATATTTGTTGTTCTGTATGGAAAACTCTGCTGAACCAGAGCAAT
CTTTGGCTTGTCAATGTTTGGTTAGAACTCCTGAAGTTGATGATGAAGCTTTGGAGAAGTTTG
ATAAAGCTTTGAAGGCTTTGCCAATGCATATCAGATTGTCTTTCAATCCTACTCAATTGGAAGA
GCAATGTCACATTCATCCACACCCTCATTTGTCTTTCATGGCTATTCCACCTAAGAAAAACCAA
GATAAGACTGAAATCCCAACTATTAATACTATTGCTTCTGGAGAGCCAACTTCTACTCCTACTAT
TGAAGCTGTTGAGTCTACTGTTGCTACTTTGGAAGCTTCTCCTGAAGTTATTGAGTCTCCACC
TGAGATTAATACTGTTCAAGTTACTTCTACTGCTGTTGGTTCTGGATCCCATCACCATCACCAT
CACTAA (SEQ ID NO:14).

Secreted BLG_(S)N-linker-CMP_(S)T were the linker is the bovine chymosin recognition and cleavage sequence.

EAEALIVTQTMKGLDIQKVAGTWYNLAMAANDINLLDAQNAPLRVYVEELKPTPEGDLEILLQKW
ENGECAQKKIIAEKTKIPAVFKIDALNENKVLVLDTDYKKYLLFCMENNAEPEQNLACQCLVRTPE
VDDEALEKFDKALKALPMHIRLNFNPTQLEEQCHIHPHPHLTFMAIPPKKNQDKTEIPTINTIATGE
PTTTPTIEAVETTVATLEATPEVIETPPEINTVQVTTTAVGGGHHHHHH (SEQ ID NO:15).

DNA sequence coding for a protein according to SEQ ID NO:15. Secretion signal, linker and tag underlined. Codon optimized for expression in *Pichia pastoris.*

ATGAGATTTCCATCTATTTTCACTGCTGTTTTGTTTGCTGCTTCTTCTGCTTTGGCTGCTCCAG
TTAACACTACTACTGAAGATGAGACTGCTCAAATTCCTGCTGAAGCTGTTATTGGTTACTCTGA
TTTGGAGGGAGATTTTGATGTTGCTGTTTTGCCTTTCTCTGCTTCTATCGCTGCTAAGGAAGA
GGGTGTTTCTTTGGAAAAGAGAGAAGCTGAGGCTTTGATTGTTACTCAAACTATGAAGGGTT
TGGATATCCAAAAGGTTGCTGGTACTTGGTACAACTTGGCTATGGCTGCTAACGATATTAACTT
GTTGGATGCTCAAAACGCTCCATTGAGAGTTTATGTTGAAGAGTTGAAACCAACTCCTGAAG
GAGATTTGGAAATTTTGTTGCAAAAGTGGGAAAACGGAGAGTGTGCTCAAAAGAAAATTATC
GCTGAAAAGACTAAGATCCCTGCTGTTTTTAAAATCGATGCTTTGAACGAGAATAAGGTTTTG
GTTTTGGATACTGATTACAAGAAATATTTGTTGTTCTGTATGGAAAACAACGCTGAACCAGAGC
AAAACTTGGCTTGTCAATGTTTGGTTAGAACTCCTGAAGTTGATGATGAAGCTTTGGAGAAGT
TTGATAAAGCTTTGAAGGCTTTGCCAATGCATATCAGATTGAACTTCAATCCTACTCAATTGGA
AGAGCAATGTCACATTCATCCACACCCTCATTTGACTTTCATGGCTATTCCACCTAAGAAAAAC
CAAGATAAGACTGAAATCCCAACTATTAATACTATTGCTACTGGAGAGCCAACTACTACTCCTA

CTATTGAAGCTGTTGAGACTACTGTTGCTACTTTGGAAGCTACTCCTGAAGTTATTGAGACTC
CACCTGAGATTAATACTGTTCAAGTTACTACTACTGCTGTTGGTGGAGGTCATCACCATCACC
ATCACTAA (SEQ ID NO:16).

Secreted BLG_(C)VA-linker-CMP were the linker is the bovine chymosin recognition and cleavage sequence.

EAEALIVTQTMKGLDIQKVAGTWYSLAMAASDISLLDAQSAPLRVYVEELKPTPEGDLEILLQKWE
NGEVAQKKIIAEKTKIPAVFKIDALNENKVLVLDTDYKKYLLFAMENSAEPEQSLAVQALVRTPEVD
DEALEKFDKALKALPMHIRLSFNPTQLEEQAHIHPHPHLSFMAIPPKKNQDKTEIPTINTIASGEPT
STPTIEAVESTVATLEASPEVIESPPEINTVQVTSTAVGSGSHHHHHH (SEQ ID NO:17).

DNA sequence coding for a protein according to SEQ ID NO: 17. Secretion signal, linker and tag underlined. Codon optimized for expression in *Pichia pastoris.*

ATGAGATTTCCATCTATTTTCACTGCTGTTTTGTTTGCTGCTTCTTCTGCTTTGGCTGCTCCAG
TTAACACTACTACTGAAGATGAGACTGCTCAAATTCCTGCTGAAGCTGTTATTGGTTACTCTGA
TTTGGAGGGAGATTTTGATGTTGCTGTTTTGCCTTTCTCTGCTTCTATCGCTGCTAAGGAAGA
GGGTGTTTCTTTGGAAAAGAGAGAAGCTGAGGCTTTGATTGTTACTCAAACTATGAAGGGTT
TGGATATCCAAAAGGTTGCTGGTACTTGGTACTCTTTGGCTATGGCTGCTTCTGATATTTCTTT
GTTGGATGCTCAATCTGCTCCATTGAGAGTTTATGTTGAAGAGTTGAAACCAACTCCTGAAGG
AGATTTGGAAATTTTGTTGCAAAAGTGGGAAAACGGAGAGGTTGCTCAAAAGAAAATTATCGC
TGAAAAGACTAAGATCCCTGCTGTTTTTAAAATCGATGCTTTGAACGAGAATAAGGTTTTGGTT
TTGGATACTGATTACAAGAAATATTTGTTGTTCGCTATGGAAAACTCTGCTGAACCAGAGCAAT
CTTTGGCTGTTCAAGCTTTGGTTAGAACTCCTGAAGTTGATGATGAAGCTTTGGAGAAGTTTG
ATAAAGCTTTGAAGGCTTTGCCAATGCATATCAGATTGTCTTTCAATCCTACTCAATTGGAAGA
GCAAGCTCACATTCATCCACACCCTCATTTGTCTTTCATGGCTATTCCACCTAAGAAAAACCA
AGATAAGACTGAAATCCCAACTATTAATACTATTGCTTCTGGAGAGCCAACTTCTACTCCTACT
ATTGAAGCTGTTGAGTCTACTGTTGCTACTTTGGAAGCTTCTCCTGAAGTTATTGAGTCTCCA
CCTGAGATTAATACTGTTCAAGTTACTTCTACTGCTGTTGGTTCTGGATCCCATCACCATCACC
ATCACTAA (SEQ ID NO:18).

Secreted BLG_(M)L-linker-CMP were the linker is the bovine chymosin recognition and cleavage sequence.

EAEALIVTQTLKGLDIQKVAGTWYSLALAASDISLLDAQSAPLRVYVEELKPTPEGDLEILLQKWE
NGECAQKKIIAEKTKIPAVFKIDALNENKVLVLDTDYKKYLLFCLENSAEPEQSLACQCLVRTPEVD
DEALEKFDKALKALPLHIRLSFNPTQLEEQCHIHPHPHLSFMAIPPKKNQDKTEIPTINTIASGEPT
STPTIEAVESTVATLEASPEVIESPPEINTVQVTSTAVGSGSHHHHHH (SEQ ID NO:19).

DNA sequence coding for a protein according to SEQ ID NO: 19. Secretion signal marked in grey, linker and tag underlined. Codon optimized for expression in *Pichia pastoris.*

ATGAGATTTCCATCTATTTTCACTGCTGTTTTGTTTGCTGCTTCTTCTGCTTTGGCTGCTCCAG
TTAACACTACTACTGAAGATGAGACTGCTCAAATTCCTGCTGAAGCTGTTATTGGTTACTCTGA
TTTGGAGGGAGATTTTGATGTTGCTGTTTTGCCTTTCTCTGCTTCTATCGCTGCTAAGGAAGA
GGGTGTTTCTTTGGAAAAGAGAGAAGCTGAGGCTTTGATTGTTACTCAAACTTTGAAGGGTT
TGGATATCCAAAAGGTTGCTGGTACTTGGTACTCTTTGGCTTTGGCTGCTTCTGATATTTCTTT
GTTGGATGCTCAATCTGCTCCATTGAGAGTTTATGTTGAAGAGTTGAAACCAACTCCTGAAGG
AGATTTGGAAATTTTGTTGCAAAAGTGGGAAAACGGAGAGTGTGCTCAAAAGAAAATTATCGC
TGAAAAGACTAAGATCCCTGCTGTTTTTAAAATCGATGCTTTGAACGAGAATAAGGTTTTGGTT
TTGGATACTGATTACAAGAAATATTTGTTGTTCTGTTTGGAAAACTCTGCTGAACCAGAGCAAT
CTTTGGCTTGTCAATGTTTGGTTAGAACTCCTGAAGTTGATGATGAAGCTTTGGAGAAGTTTG
ATAAAGCTTTGAAGGCTTTGCCATTGCATATCAGATTGTCTTTCAATCCTACTCAATTGGAAGA
GCAATGTCACATTCATCCACACCCTCATTTGTCTTTCATGGCTATTCCACCTAAGAAAAACCAA
GATAAGACTGAAATCCCAACTATTAATACTATTGCTTCTGGAGAGCCAACTTCTACTCCTACTAT
TGAAGCTGTTGAGTCTACTGTTGCTACTTTGGAAGCTTCTCCTGAAGTTATTGAGTCTCCACC
TGAGATTAATACTGTTCAAGTTACTTCTACTGCTGTTGGTTCTGGATCCCATCACCATCACCAT
CACTAA (SEQ ID NO:20).

### Example 3: in vivo testing of recombinant proteins

### Xenograft experiment

1. 5-10 mice per experimental group are grown to adequate age under standard conditions. The race of mouse will be selected by the skilled person according to the cancer line to be tested (e.g. CD-1 female nude mice can be used with DLD1 or HCT116 cells, Balb/c CAnN.Cg-Foxn1nu/Crl mice can be used with SNU-398 cells). The "adequate age" can be determined by the skilled person depending on mouse race (e.g. 7-9 weeks old for CD-1, 4-6 week old for Balb/c).

Experimental groups in one experiment are:
A: Standard diet
B: Standard diet + chemotherapy
C: AA restriction diet based on free AA
D: AA restriction diet based on free AA + chemotherapy
E: AA restriction diet based on recombinant protein
F: AA restriction diet based on recombinant protein + chemotherapy

The chemotherapy will be selected by the skilled person according to the cell line (e.g. PH-755 is used on DLD1 or HCT116 cells, ABT-2663 is used on SNU-398 cells).

2. Cells from a defined cancer line are grown under standard conditions.

The cell line will be selected by the skilled person according to the AA restriction treatment to be tested (e.g. DLD1 or HCT116 cells are used to test Serine/Glycine restriction diets, ABT-2663 is used to test Arginine restriction diets).

3. Unilateral subcutaneous injection of the cells from step 2 is applied to the mice from step 1. For example, 2 × 10⁶ HCT116 or SNU-398 cells or 4 × 10⁶ DLD-1 cells suspended in 100 µl PBS can be injected.

4. For a number of days depending on the cell line (e.g. 10 days for HCT116 or 2 days for DLD-1) the mice are kept on standard conditions (standard diet, no chemotherapy). After this period the mice are shifted to the diet corresponding with their experimental group. Other experimental designs are possible where the mice are shifted to the experiential diets a number of days before the xenograft implantation (e.g. 1 week before injection of SNU-398 cells).

5. At the beginning of the experiment (e.g. SNU-398 cells) or a number of days after shift to experimental-group diet (e.g. 4 days for HCT116 or 2 days for DLD-1) mice are given a dose of either chemotherapy or vehicle.

6. The diet is maintained and further doses of chemotherapy are applied (e.g. PH-755: daily dose, 100 mg/kg for 7 days, followed by 50 mg/kg for 6 days, followed by 75 mg/kg for 6 days; ABT-2663 50 mg/kg 5 times a week).

7. Subcutaneous growth is measured two to three times a week with a caliper. The volume of the tumour is calculated as length × width²/2 and used as a marker of the efficacy of treatment.
References: Tajan, M. Nat Commun 2021 12(1):366
Missiaen, R. et al. Cell Metab 2022 34(8):1151-1167
Maddocks, O.D.K. et al. Nature 2017 544(7650):372-376
Hirakawa, D.A. et al. Nutr Res 1984 4(5):891-895

The invention may be described by any of the following clauses:
1. A recombinant protein for use in the treatment of a proliferative disease or disorder in a subject, wherein the recombinant protein comprises at least one amino acid substitution or deletion relative to a corresponding native protein, wherein the recombinant protein is not a hormone, an enzyme or an antibody.
2. The recombinant protein for use according to clause 1, wherein each residue of a first amino acid that is present in the corresponding native protein has been independently deleted or substituted for another amino acid in the recombinant protein.
3. The recombinant protein for use according to any preceding clause, wherein the recombinant protein comprises all nine essential amino acids.
4. The recombinant protein for use according to clause 3, wherein the recombinant protein comprises at least 10 wt % (e.g. at least 20, 30 or 40 wt%) essential amino acids.
5. The recombinant protein for use according to any one of clauses 2 to 4, wherein the first amino acid is selected from: arginine, asparagine, cysteine, glycine, glutamine, leucine, methionine, phenylalanine, proline, serine, threonine, tyrosine and valine.
6. The recombinant protein for use according to clause 5, wherein:
   (i) the first amino acid is arginine and each arginine residue present in the corresponding native protein has been independently replaced by lysine or glutamine;
   (ii) the first amino acid is asparagine and each asparagine residue present in the corresponding native protein has been independently replaced by aspartate or serine;
   (iii) the first amino acid is cysteine and each cysteine residue present in the corresponding native protein has been independently replaced by alanine or valine;
   (iv) the first amino acid is glycine and each glycine residue present in the corresponding native protein has been independently replaced by alanine;
   (v) the first amino acid is glutamine and each glutamine residue present in the corresponding native protein has been independently replaced by glutamate or arginine;
   (vi) the first amino acid is leucine and each leucine residue present in the corresponding native protein has been independently replaced by isoleucine, methionine, or valine;
   (vii) the first amino acid is methionine and each methionine residue present in the corresponding native protein has been independently replaced by isoleucine or leucine;
   (vii) the first amino acid is phenylalanine and each phenylalanine residue present in the corresponding native protein has been independently replaced by tyrosine;
   (vii) the first amino acid is proline and each proline residue present in the corresponding native protein has been independently replaced by alanine;
   (viii) the first amino acid is serine and each proline residue present in the corresponding native protein has been independently replaced by asparagine or threonine;
   (ix) the first amino acid is threonine and each threonine residue present in the corresponding native protein has been independently replaced by serine;
   (x) the first amino acid is tyrosine and each tyrosine residue present in the corresponding native protein has been independently replaced by phenylalanine;
   (xi) the first amino acid is valine and each valine residue present in the corresponding native protein has been independently replaced by isoleucine or leucine
   (xii) the first amino acid is glycine and each glycine residue present in the corresponding native protein has been independently replaced by threonine;
   (xiii) the first amino acid is glycine and each glycine residue present in the corresponding native protein has been independently replaced by asparagine; or
   (xiv) the first amino acid is glycine and each glycine residue present in the corresponding native protein has been independently replaced by threonine, lysine or aspartate.
7. The recombinant protein for use according to any preceding clause, wherein the recombinant protein has at least 80% (e.g. at least 90%) sequence identity to the corresponding native protein.
8. The recombinant protein for use according to any preceding clause, wherein the protein is a contractile, structural, storage or transport protein.
9. The recombinant protein for use according to any preceding clause, wherein the recombinant protein is a storage protein, optionally wherein the storage protein is an animal storage protein (e.g. a milk protein) or a plant storage protein.
10. The recombinant protein for use according to clause 9, wherein the animal storage protein is a milk protein, optionally wherein the milk protein is selected from casein (e.g. α-S1 casein, α-S2 casein, β-casein and kappa casein), lactalbumin (e.g. alpha-lactalbumin (LALBA)) or lactoglobulin (e.g. beta lactoglobulin (BLG)).
11. The recombinant protein for use according to clause 9, wherein the animal storage protein is an egg protein, optionally wherein the egg protein is selected from ovalbumin, ovaltransferrin, ovamucoid, avidin, biotin, high-density lipoprotein (HDL), low-density lipoprotein (LDL), phosvitin, livetin, globulin, and ovomucin.
12. The recombinant protein for use according to clause 9, wherein the plant storage protein is a protein derived from soy, wheat, maize, barley, rye, buckwheat, millet, oat, sorghum, pea, rice, cacao, oil seeds, cereals, legumes, nuts, lentil, chickpea, peanut, almond, coconut, algae, spirulina, quinoa, sunflower seed, canola seed, or hemp seed.
13. The recombinant protein for use according to clause 9, wherein the plant storage protein is selected from glycinin (e.g. glycinin G1, G2, G3, G4 or G5), conglycinin (e.g. beta-conglycinin alpha 1, beta-conglycinin beta 1, beta-conglycinin alpha 2, or beta-conglycinin beta 2), gliadin (e.g. alpha-gliadin, beta-gliadin, gamma-gliadin), avenin-like a1, avenin-like b1, hordein (e.b. B-1 hordein, B-3 hordein, C hordein, or gamma hordein 1), Glutelin-2, 22 kDa alpha-zein 16, 22 kDa alpha-zein 14, alpha kafirin, avenin (e.g. avenin-3, avenin-E), vicilin, albumin (e.g. albumin-1a, albumin-2), or glutelin (e.g. glutelin type I or type-B 2).
14. The recombinant protein for use according to clause 9, wherein the recombinant protein is selected from: casein, beta-lactoglobulin, ovalbumin, hordein, conglycinin or alpha-lactalbumin.
15. The recombinant protein for use according to any preceding claim, wherein the proliferative disease is cancer.
16. The recombinant protein for use according to clause 15, wherein the cancer is selected from: brain cancer (including glioblastoma, glioma and medullablastoma), bladder cancer (e.g. bladder melanoma), liver cancer (e.g. hepatocellular carcinoma), melanoma, colon cancer, colorectal cancer, prostate cancer, breast cancer, leukemia, ovarian cancer, kidney cancer, lung cancer (e.g. lung carcinoma), pancreatic cancer, uterine cancer, and head and neck cancer.
17. The recombinant protein for use according to any one of clauses 2 to 16, wherein the subject is maintained on a diet which is substantially deficient in the first amino acid.
18. The recombinant protein for use according to any preceding clause, wherein the treatment results in a reduced rate of cancerous cell proliferation, tumor growth and/or reduced metastases.
19. The recombinant protein for use according to any preceding clause, wherein the treatment further comprises surgery, radiotherapy and/or administration of a further therapeutic agent, optionally wherein the further therapeutic agent is a chemotherapeutic agent.
20. A recombinant protein comprising at least one amino acid substitution relative to a corresponding wild-type protein, wherein each residue of a first type of amino acid that is present in the corresponding native protein has been independently deleted or substituted for another amino acid in the recombinant protein, wherein the recombinant protein is a storage protein.
21. A recombinant protein according to clause 20, wherein the recombinant protein is fused to a caseinomacropeptide (CMP), or a fragment thereof, forming a fusion protein.
22. The recombinant protein of clause 21, wherein the fusion protein does not comprise a full-length kappa casein protein.
23. The recombinant protein of clause 21 or clause 22, wherein the CMP or fragment thereof comprises a sequence of no more than 150, no more than 120, no more than 100, no more than 90, no more than 80, no more than 75, no more than 70, no more than 65 or no more than 60 consecutive amino acids derived from the C-terminus of kappa casein.
24. The recombinant protein of any one of clauses 21 to 23, wherein the CMP or fragment thereof comprises an amino acid sequence of at least 20 residues.
25. The recombinant protein of any one of clauses 21 to 24, wherein the CMP or fragment thereof comprises:
   (i) the amino acid sequence:
      X3SGX2PX1 X1X1 PTX3X2X3X3X2S X1X3X3X1X3X2X3X1 PX2 X3 (SEQ ID NO:5) wherein
      each X1 is independently selected from S and T,
      each X2 is independently selected from D and E, and
      each X3 is independently selected from A, I, L and V, or
   (ii) an amino acid sequence which is at least 80% identical to SEQ ID NO: 5, optionally wherein at least 3 of the X1 residues are retained.
26. The recombinant protein of any one of clauses 21 to 25, wherein the CMP or fragment thereof comprises
   (i) an amino acid sequence selected from: 
X3SGX2PX1 X1X1PTX3X2X3X3X2S X1X3X3X1X3X2X3X1PX2 X3X3X2SPPX2X3NT
X3QX3X1STX3X3 (SEQ ID NO:6);
 and 
KNQX2KTX2X3PX1 X3NTX3X3SGX2PX1 X1X1PTX3X2X3X3X2S X1X3X3X1X3X2X3X1PX2
X3 (SEQ ID NO:7),
 or
   (ii) an amino acid sequence which is at least 80% identical to SEQ ID NO:6, optionally wherein at least 3 of the X1 residues are retained, or
   (iii) an amino acid sequence which is at least 80% identical to SEQ ID NO:7, optionally wherein at least 3 of the X1 residues are retained.
27. The recombinant protein of any one of clauses 21 to 26, wherein the CMP or fragment thereof comprises
   (i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:8);
 or
   (ii) an amino acid sequence which is at least 80% identical to SEQ ID NO:8, optionally wherein at least 3 of the X1 residues are retained.
28. The recombinant protein of part (ii) or (iii) of any one of clauses 25 to 27, wherein all of the X1 residues are retained.
29. The recombinant protein of any one of clauses 21 to 28, wherein the CMP or fragment thereof comprises
   (i) the amino acid sequence
      ASGEPT STPTIEAVES TVATLEASPE V (SEQ ID NO:9), or
   (ii) an amino acid sequence which is at least 80% identical to SEQ ID NO: 9.
30. The recombinant protein of any one of clauses 21 to 29, wherein the CMP or fragment thereof comprises:
   (i) an amino acid sequence selected from:
      ASGEPT STPTIEAVES TVATLEASPE VIESPPEINT VQVTSTAV (SEQ ID NO:10);
      KNQDKTEIPT INTIASGEPT STPTIEAVES TVATLEASPE V (SEQ ID NO:11); and
      KNQDKTEIPT INTIASGEPT STPTIEAVES TVATLEASPE VIESPPEINT VQVTSTAV (SEQ ID NO:12), or
   (ii) an amino acid sequence which is at least 80% identical to SEQ ID NO:10, SEQ ID NO:11 or SEQ ID NO:12.
31. The recombinant protein of any one of clauses 21 to 30, wherein each residue of the first amino acid that is present in a corresponding native CMP is substituted by another amino acid in the CMP or fragment thereof fused to the recombinant protein.
32. The recombinant protein of clause 31, wherein each serine residue present in a corresponding native CMP is substituted by another amino acid in the CMP or fragment thereof, optionally wherein each serine residue is substituted by threonine.
33. The recombinant protein of any one of clauses 21 to 30, wherein each glycine residue present in a corresponding native CMP is substituted by another amino acid in the CMP or fragment thereof, optionally wherein each glycine residue is substituted by alanine.
34. The recombinant protein of any one of clauses 21 to 33, which further comprises a linker between the recombinant protein and the CMP
35. The recombinant protein of clause 34, wherein the linker comprises a cleavage site, optionally an enzyme cleavage site.
36. The recombinant protein of clause 35, wherein the enzyme cleavage site is a protease cleavage site, optionally a chymosin cleavage site.
37. The recombinant protein of any one of clauses 21 to 35, wherein the fusion protein is devoid of the first amino acid.
38. A composition comprising the recombinant protein according to any one of clauses 20 to 37.
39. A composition according to clause 38, wherein the composition is a food composition.
40. A nucleic acid comprising a sequence encoding a recombinant protein according to clause 39.
41. A vector comprising the nucleic acid molecule according to clause 40.
42. A host cell comprising the nucleic acid molecule according to clause 40, or the vector according to clause 41.
43. A method of producing a recombinant protein, the method comprising culturing the host cell according to clause 42 under conditions suitable for expression of the recombinant protein.
44. A method of preparing a recombinant protein for the treatment of a subject with a proliferative disease, the method comprising:
   - expressing in a host cell a recombinant protein which is devoid of at least one amino acid; and
   - isolating the recombinant protein from the host cell.
45. The method according to clause 44, wherein the method further comprises purifying the isolated recombinant protein.
46. The method according to clause 44 or 45, wherein the method further comprises identifying an amino acid which modulates the proliferation and/or the survival of cells associated with the disease (e.g. cancer or tumour cells).
47. The method according to clause 46, wherein identifying an amino acid which modulates the proliferation and/or survival of cells associated with the disease is carried out using metabolomic and/or genomic analysis.
48. The method according to clause 46 or 47, wherein identifying the amino acid which modulates the proliferation and/or survival of cells associated with the disease is carried out using a machine learning model.
49. The method according to any one of clauses 44 to 48, wherein the method further comprises preparing a nucleic acid molecule comprising a sequence encoding the recombinant protein.
50. The method according to clause 49, wherein the method further comprises introducing the nucleic acid molecule into the host cell.

## Claims

1. A recombinant protein for use in the treatment of a proliferative disease or disorder in a subject, wherein the recombinant protein comprises at least one amino acid substitution or deletion relative to a corresponding native protein, wherein the recombinant protein is not a hormone, an enzyme or an antibody.

2. The recombinant protein for use according to claim 1, wherein each residue of a first amino acid that is present in the corresponding native protein has been independently deleted or substituted for another amino acid in the recombinant protein.

3. The recombinant protein for use according to claim 1 or claim 2, wherein the proliferative disease is cancer, optionally wherein the cancer is selected from: brain cancer (including glioblastoma, glioma and medullablastoma), bladder cancer (e.g. bladder melanoma), liver cancer (e.g. hepatocellular carcinoma), melanoma, colon cancer, colorectal cancer, prostate cancer, breast cancer, leukemia, ovarian cancer, kidney cancer, lung cancer (e.g. lung carcinoma), pancreatic cancer, uterine cancer, and head and neck cancer.

4. The recombinant protein for use according to any preceding claim, wherein the subject is maintained on a diet which is substantially deficient in the first amino acid, optionally wherein the treatment further comprises surgery, radiotherapy and/or administration of a further therapeutic agent.

5. A recombinant protein comprising at least one amino acid substitution relative to a corresponding wild-type protein, wherein each residue of a first type of amino acid that is present in the corresponding native protein has been independently deleted or substituted for another amino acid in the recombinant protein, wherein the recombinant protein is a storage protein.

6. The recombinant protein for use according to any one of claims 1 to 4, or the recombinant protein according to claim 5, wherein the recombinant protein comprises all nine essential amino acids, optionally wherein the recombinant protein comprises at least 10 wt % essential amino acids.

7. The recombinant protein for use according to any one of claims 1 to 4 and 6, or the recombinant protein according to claim 5 or claim 6, wherein the first amino acid is selected from: arginine, asparagine, cysteine, glycine, glutamine, leucine, methionine, phenylalanine, proline, serine, threonine, tyrosine and valine.

8. The recombinant protein for use according to any one of claims 1 to 4, 6 and 7, or the recombinant protein according to any one of claims 5 to 7, wherein the protein is a contractile, structural, storage or transport protein, optionally wherein the recombinant protein is: (A) an animal storage protein, further optionally wherein
(i) the animal storage protein is a milk protein selected from casein (e.g. α-S1 casein, α-S2 casein, β-casein and kappa casein), lactalbumin (e.g. alpha-lactalbumin (LALBA)) or lactoglobulin (e.g. beta lactoglobulin (BLG)); or
(ii) the animal storage protein is an egg protein selected from ovalbumin, ovaltransferrin, ovamucoid, avidin, biotin, high-density lipoprotein (HDL), low-density lipoprotein (LDL), phosvitin, livetin, globulin, and ovomucin; or
(B) a plant storage protein, further optionally wherein
(i) the plant storage protein is derived from soy, wheat, maize, barley, rye, buckwheat, millet, oat, sorghum, pea, rice, cacao, oil seeds, cereals, legumes, nuts, lentil, chickpea, peanut, almond, coconut, algae, spirulina, quinoa, sunflower seed, canola seed, or hemp seed; or
(ii) the plant storage protein is selected from glycinin (e.g. glycinin G1, G2, G3, G4 or G5), conglycinin (e.g. beta-conglycinin alpha 1, beta-conglycinin beta 1, beta-conglycinin alpha 2, or beta-conglycinin beta 2), gliadin (e.g. alpha-gliadin, beta-gliadin, gamma-gliadin), avenin-like a1, avenin-like b1, hordein (e.b. B-1 hordein, B-3 hordein, C hordein, or gamma hordein 1), Glutelin-2, 22 kDa alpha-zein 16, 22 kDa alpha-zein 14, alpha kafirin, avenin (e.g. avenin-3, avenin-E), vicilin, albumin (e.g. albumin-1a, albumin-2), and glutelin (e.g. glutelin type I ortype-B 2).

9. The recombinant protein for use according to claim 8 or the recombinant protein according to claim 8, wherein the recombinant protein is selected from: casein, beta-lactoglobulin, ovalbumin, hordein, conglycinin or alpha-lactalbumin.

10. A recombinant protein according to any one of claims 5 to 9, wherein the recombinant protein is fused to a caseinomacropeptide (CMP).

11. A composition comprising the recombinant protein according to claim 9 or claim 10.

12. A nucleic acid comprising a sequence encoding a recombinant protein according to any one of claims 5 to 10.

13. A vector comprising the nucleic acid molecule according to claim 12.

14. A host cell comprising the nucleic acid molecule according to claim 12, or the vector according to claim 13.

15. A method of preparing a recombinant protein for the treatment of a subject with a proliferative disease, the method comprising:
- expressing in a host cell a recombinant protein which is devoid of at least one amino acid; and
- isolating the recombinant protein from the host cell, optionally wherein the method further comprises:
(i) purifying the isolated recombinant protein;
(ii) identifying an amino acid which modulates the proliferation and/or the survival of cells associated with the disease (e.g. cancer or tumour cells), further optionally wherein identifying an amino acid which modulates the proliferation and/or survival of cells associated with the disease is carried out using metabolomic and/or genomic analysis, and/or using a machine learning model;
(iii) preparing a nucleic acid molecule comprising a sequence encoding the recombinant protein; and/or
(iv) introducing the nucleic acid molecule into the host cell.
